# EUROPEAN PATENT APPLICATION

(11) **EP 4 227 301 A1**
(43) Date of publication of application: **16.08.2023**
(21) Application number: 21877853.8
(22) Date of filing: 02.09.2021
(51) Int. Cl.: C07D 403/04, C07D 409/14, C07D 403/14, C07D 495/04, C07D 403/10, H01L 51/00, H01L 51/50

(54) **HETEROCYCLIC COMPOUND AND ORGANIC LIGHT-EMITTING DEVICE COMPRISING SAME**

(30) Priority: 08.10.2020 KR 20200130104
(71) Applicant: LT Materials Co., Ltd., Yongin-si, Gyeonggi-do 17118 (KR)
(72) Inventor: LEE, Young-Jin, Yongin-si, Gyeonggi-do 17118 (KR); KWON, Su-Jin, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR); PARK, Chan-Jun, Yongin-si, Gyeonggi-do 17118 (KR)
(74) Representative: Goddar, Heinz J.
(86) International application number: PCT/KR2021/011844
(87) International publication number: WO 2022/075601

(57) **Abstract**

The present specification relates to a heterocyclic compound represented by Chemical Formula 1, and an organic light emitting device comprising the same.

## Description

### [Technical Field]

This application claims priority to and the benefits of Korean Patent Application No. 10-2020-0130104, filed with the Korean Intellectual Property Office on October 8, 2020, the entire contents of which are incorporated herein by reference.

The present specification relates to a heterocyclic compound, and an organic light emitting device comprising the same.

### [Background Art]

An organic electroluminescent device is one type of selfemissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a hostdopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent No. 4,356,429

### [Disclosure]

### [Technical Problem]

The present application relates to a heterocyclic compound, and an organic light emitting device comprising the same.

### [Technical Solution]

One embodiment of the present application provides a heterocyclic compound represented by the following Chemical Formula 1.

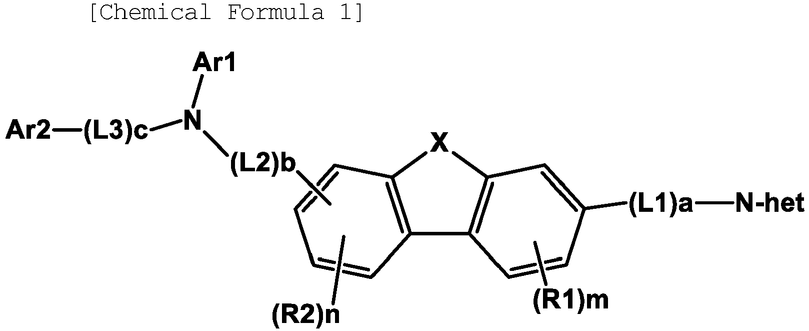

In Chemical Formula 1,
R1 and R2 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and - SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
N-het is a monocyclic or polycyclic C2 to C60 heterocyclic group substituted or unsubstituted and comprising one or more Ns,
X is O; or S,
L1 to L3 are the same as or different from each other, and each independently a direct bond; a substituted or unsubstituted C6 to C60 arylene group; or a substituted or unsubstituted C2 to C60 heteroarylene group,
Ar1 and Ar2 are the same as or different from each other, and each independently a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - NR201R202; -P(=O)RR'; or -SiRR'R",
a to c are an integer of 0 to 4,
m and n are an integer of 0 to 3,
when a to c, m and n are 2 or greater, substituents in the parentheses are the same as or different from each other, and
R201, R202, R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise one or more types of the heterocyclic compound represented by Chemical Formula 1.

### [Advantageous Effects]

A compound described in the present specification can be used as a material of an organic material layer of an organic light emitting device. The compound is capable of performing a role of a hole injection material, a hole transfer material, a light emitting material, an electron transfer material, an electron injection material, an electron blocking material, a hole blocking material or the like in an organic light emitting device. Particularly, the compound can be used as a light emitting material of an organic light emitting device.

The heterocyclic compound of the present disclosure is a compound using a core structure of dibenzofuran or dibenzothiophene, and by a No. 3 position of one side benzene ring of the dibenzofuran or dibenzothiophene being substituted with a heterocyclic group represented by N-het and the other side benzene ring being substituted with an amine-based substituent, excellent properties as a bipolar host are obtained.

Specifically, when the core structure of the heterocyclic compound according to the present application is substituted with amine, a heteroatom of the amine group enhances a hole injection ability of a hole transfer group, which increases efficiency and lifetime.

### [Description of Drawings]

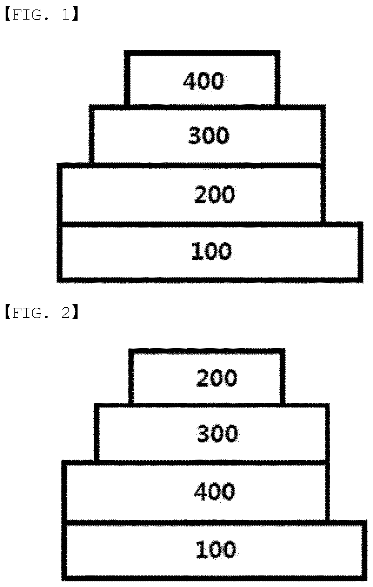

FIG. 1 to FIG. 3 are diagrams each schematically illustrating a lamination structure of an organic light emitting device according to one embodiment of the present application.

### [Reference Numeral]

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium unlike a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 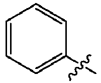 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

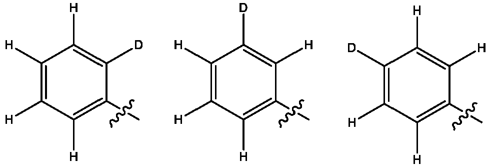

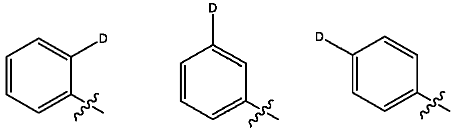

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The number of carbon atoms of the aryl group may be from 6 to 60, specifically from 6 to 40 and more specifically from 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused ring group thereof, and the like, but are not limited thereto.

In the present specification, a fluorenyl group may be substituted, and adjacent substituents may bond to each other to form a ring.

When the fluorenyl group is substituted, the following structures and the like may be included, however, the structure is not limited thereto.

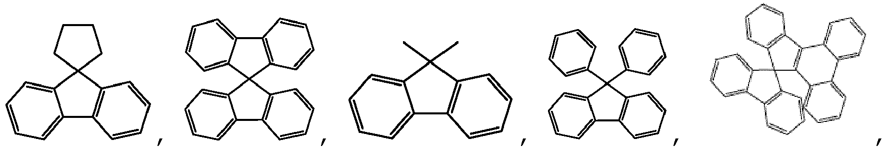

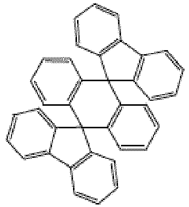

In the present specification, the heteroaryl group comprises S, O, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; and -NRR', or being unsubstituted, or being substituted with a substituent linking two or more substituents selected from among the substituents illustrated above, or being unsubstituted, and

R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

One embodiment of the present application provides a compound represented by Chemical Formula 1.

In one embodiment of the present application, R1 and R2 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and - SiRR'R", or two or more groups adjacent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R1 and R2 are the same as or different from each other, and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - P(=O)RR'; and -SiRR'R".

In another embodiment, R1 and R2 are the same as or different from each other, and may be each independently selected from the group consisting of hydrogen; deuterium; halogen; a cyano group; a substituted or unsubstituted C1 to C40 alkyl group; a substituted or unsubstituted C2 to C40 alkenyl group; a substituted or unsubstituted C2 to C40 alkynyl group; a substituted or unsubstituted C1 to C40 alkoxy group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; - P(=O)RR'; and -SiRR'R".

In another embodiment, R1 and R2 are the same as or different from each other, and may be each independently selected from the group consisting of hydrogen; deuterium; halogen; a cyano group; a substituted or unsubstituted C1 to C40 alkyl group; a substituted or unsubstituted C6 to C40 aryl group; a substituted or unsubstituted C2 to C40 heteroaryl group; -P(=O)RR'; and -SiRR'R".

In another embodiment, R1 and R2 are the same as or different from each other, and may be each independently selected from the group consisting of hydrogen; deuterium; halogen; a cyano group; a C1 to C40 alkyl group; a C6 to C40 aryl group; a C2 to C40 heteroaryl group; -P(=O)RR'; and - SiRR'R".

In another embodiment, R1 and R2 may be hydrogen.

In one embodiment of the present application, X may be O.

In one embodiment of the present application, X may be S.

In one embodiment of the present application, Chemical Formula 1 may be represented by any one of the following Chemical Formulae 2 to 5.

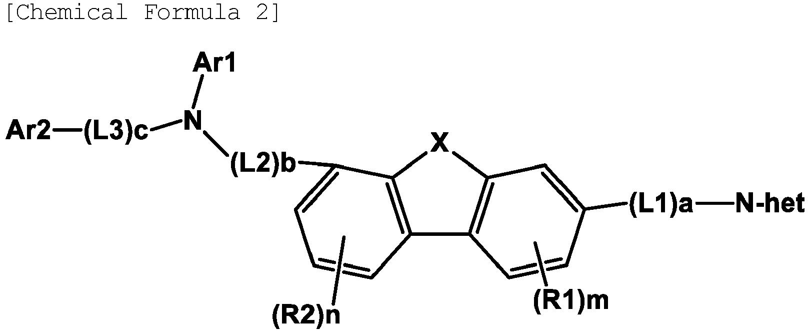

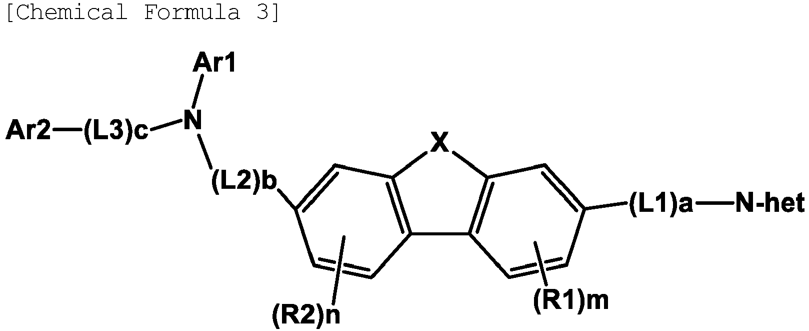

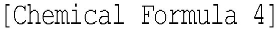

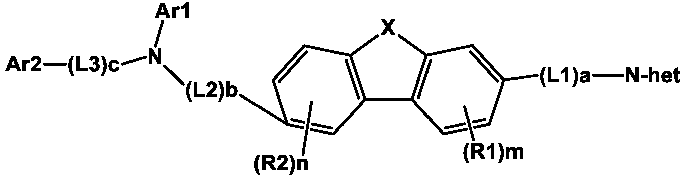

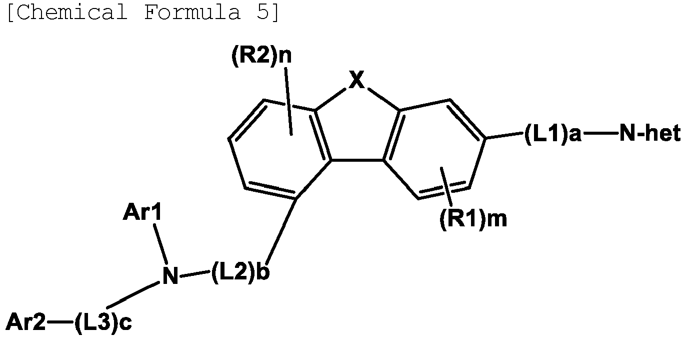

In Chemical Formulae 2 to 5,
each substituent has the same definition as in Chemical Formula 1.

In one embodiment of the present application, N-het may be a monocyclic or polycyclic C2 to C60 heterocyclic group substituted or unsubstituted and comprising one or more Ns.

In another embodiment, N-het may be a monocyclic or polycyclic C2 to C60 heterocyclic group substituted or unsubstituted and comprising one or more and three or less Ns.

In another embodiment, N-het may be a monocyclic or polycyclic C2 to C40 heterocyclic group substituted or unsubstituted and comprising one or more and three or less Ns.

In another embodiment, N-het may be a monocyclic or polycyclic C2 to C40 heterocyclic group substituted or unsubstituted and comprising one or more and three or less Ns.

In another embodiment, N-het may be a monocyclic or polycyclic C2 to C40 heterocyclic group substituted or unsubstituted and comprising one or more and three or less =N-bonds.

In another embodiment, N-het may be a monocyclic or polycyclic C2 to C40 heterocyclic group substituted or unsubstituted and comprising one or more and three or less Ns linked by a double bond.

In one embodiment of the present application, in another embodiment, N-het may be a monocyclic or polycyclic C2 to C40 heterocyclic group unsubstituted or substituted with one or more substituents selected from the group consisting of a C6 to C40 aryl group; and a C2 to C40 heteroaryl group, and comprising one or more and three or less Ns.

In another embodiment, N-het may be a monocyclic or polycyclic C2 to C40 heterocyclic group unsubstituted or substituted with one or more substituents selected from the group consisting of a C6 to C40 aryl group; and a C2 to C40 heteroaryl group, and comprising one or more and three or less =N- bonds.

In another embodiment, N-het may be a monocyclic or polycyclic C2 to C40 heterocyclic group unsubstituted or substituted with one or more substituents selected from the group consisting of a C6 to C40 aryl group; and a C2 to C40 heteroaryl group, and comprising one or more and three or less Ns linked by a double bond.

In one embodiment of the present application, N-het may be a substituted or unsubstituted pyrimidine group; a substituted or unsubstituted triazine group; a substituted or unsubstituted quinazoline group; a substituted or unsubstituted benzo[4,5]thieno[3,2-d]pyrimidine group; a substituted or unsubstituted phenanthroline group; or a substituted or unsubstituted quinoxaline group.

In one embodiment of the present application, N-het may be a pyrimidine group unsubstituted or substituted with one or more substituents selected from the group consisting of a phenyl group, a biphenyl group, a naphthyl group and a dibenzofuran group; a triazine group unsubstituted or substituted with one or more substituents selected from the group consisting of a phenyl group, a biphenyl group, a naphthyl group, a dimethylfluorenyl group, a dibenzothiophene group, a carbazole group and a dibenzofuran group; a quinazoline group unsubstituted or substituted with a phenyl group, a biphenyl group or a naphthyl group; a benzo[4,5]thieno[3,2-d]pyrimidine group unsubstituted or substituted with a phenyl group or a naphthyl group; a phenanthroline group unsubstituted or substituted with a phenyl group; or a quinoxaline group unsubstituted or substituted with a phenyl group.

In one embodiment of the present application, N-het may be substituted again with a naphthyl group; or a carbazole group.

In one embodiment of the present application, N-het may be represented by any one of the following Chemical Formulae 1-1 to 1-5.

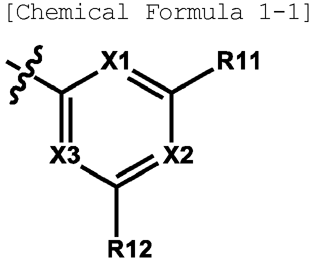

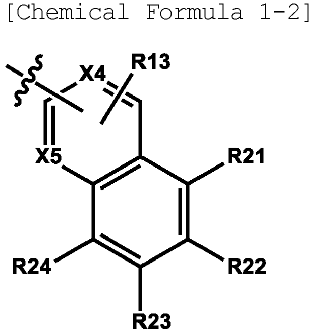

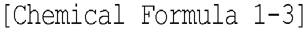

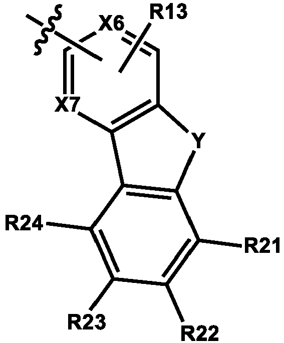

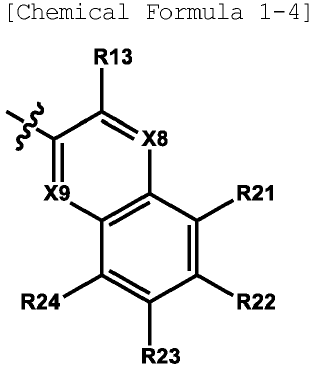

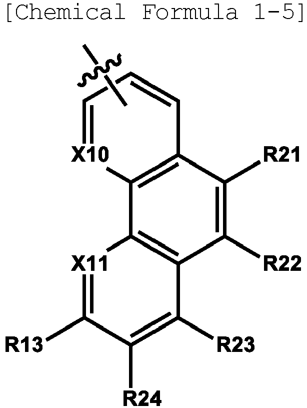

In Chemical Formulae 1-1 to 1-5,
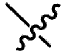 means a position linked to Chemical Formula 1,
X1 to X3 are the same as or different from each other, and each independently N or CRa,
X4 and X5 are the same as or different from each other, and each independently N or CRa,
X6 and X7 are the same as or different from each other, and each independently N or CRa,
X8 and X9 are the same as or different from each other, and each independently N or CRa,
X10 and X11 are the same as or different from each other, and each independently N or CRa,
at least one of X1 to X3, at least one of X6 and X7, at least one of X8 and X9 and at least one of X10 and X11 are N,
Y is O; or S,
R11 to R13 are the same as or different from each other, and each independently hydrogen; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
R21 to R24 are the same as or different from each other, and each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
Ra is hydrogen; a substituted or unsubstituted C6 to C60 aryl group; or a substituted or unsubstituted C2 to C60 heteroaryl group.

In one embodiment of the present application, R11 to R13 are the same as or different from each other, and may be each independently hydrogen; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another embodiment, R11 to R13 are the same as or different from each other, and may be each independently hydrogen; a substituted or unsubstituted C6 to C60 aryl group; or a substituted or unsubstituted C2 to C60 heteroaryl group.

In another embodiment, R11 to R13 are the same as or different from each other, and may be each independently hydrogen; a substituted or unsubstituted C6 to C40 aryl group; or a substituted or unsubstituted C2 to C40 heteroaryl group.

In another embodiment, R11 to R13 are the same as or different from each other, and may be each independently hydrogen; a C6 to C40 aryl group unsubstituted or substituted with a C1 to C40 alkyl group, a C6 to C40 aryl group or a C2 to C40 heteroaryl group; or a C2 to C40 heteroaryl group unsubstituted or substituted with a C6 to C40 aryl group.

In another embodiment, R11 to R13 are the same as or different from each other, and may be each independently a C6 to C40 aryl group unsubstituted or substituted with a C1 to C40 alkyl group, a C6 to C40 aryl group or a C2 to C40 heteroaryl group; or a C2 to C40 heteroaryl group unsubstituted or substituted with a C6 to C20 aryl group.

In another embodiment, R11 to R13 are the same as or different from each other, and may be each independently a phenyl group unsubstituted or substituted with a carbazole group or a naphthyl group; a biphenyl group; a naphthyl group; a terphenyl group; a dimethylfluorenyl group; a carbazole group unsubstituted or substituted with a phenyl group; a dibenzofuran group; or a dibenzothiophene group.

In one embodiment of the present application, R21 to R24 are the same as or different from each other, and may be each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another embodiment, R21 to R24 are the same as or different from each other, and may be each independently hydrogen; deuterium; a substituted or unsubstituted C6 to C60 aryl group; or a substituted or unsubstituted C2 to C60 heteroaryl group.

In another embodiment, R21 to R24 are the same as or different from each other, and may be each independently hydrogen; deuterium; a substituted or unsubstituted C6 to C40 aryl group; or a substituted or unsubstituted C2 to C40 heteroaryl group.

In another embodiment, R21 to R24 are the same as or different from each other, and may be each independently hydrogen; deuterium; a C6 to C40 aryl group; or a C2 to C40 heteroaryl group.

In another embodiment, R21 to R24 are the same as or different from each other, and may be each independently hydrogen; or deuterium.

In one embodiment of the present application, Ra may be hydrogen.

In one embodiment of the present application, L1 to L3 are the same as or different from each other, and may be each independently a direct bond; a substituted or unsubstituted C6 to C60 arylene group; or a substituted or unsubstituted C2 to C60 heteroarylene group.

In another embodiment, L1 to L3 are the same as or different from each other, and may be each independently a direct bond; a substituted or unsubstituted C6 to C40 arylene group; or a substituted or unsubstituted C2 to C40 heteroarylene group.

In another embodiment, L1 to L3 are the same as or different from each other, and may be each independently a direct bond; or a substituted or unsubstituted C6 to C40 arylene group.

In another embodiment, L1 to L3 are the same as or different from each other, and may be each independently a direct bond; or a C6 to C40 arylene group.

In another embodiment, L1 to L3 are the same as or different from each other, and may be each independently a direct bond; or a monocyclic or polycyclic C6 to C40 arylene group.

In another embodiment, L1 to L3 are the same as or different from each other, and may be each independently a direct bond; a monocyclic C6 to C10 arylene group; or a polycyclic C10 to C40 arylene group.

In another embodiment, L1 to L3 are the same as or different from each other, and may be each independently a direct bond; or a phenylene group.

In one embodiment of the present application, Ar1 and Ar2 are the same as or different from each other, and may be each independently a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - NR201R202; -P(=O)RR'; or -SiRR'R".

In another embodiment, Ar1 and Ar2 are the same as or different from each other, and may be each independently a substituted or unsubstituted C6 to C60 aryl group; a substituted or unsubstituted C2 to C60 heteroaryl group; - NR201R202; -P(=O)RR'; or -SiRR'R".

In another embodiment, Ar1 and Ar2 are the same as or different from each other, and may be each independently a substituted or unsubstituted C6 to C40 aryl group; a substituted or unsubstituted C2 to C40 heteroaryl group; or - NR201R202.

In another embodiment, Ar1 and Ar2 are the same as or different from each other, and may be each independently a C6 to C40 aryl group unsubstituted or substituted with a C1 to C40 alkyl group or a C6 to C40 aryl group; a C2 to C40 heteroaryl group unsubstituted or substituted with a C6 to C40 aryl group; or -NR201R202.

In another embodiment, Ar1 and Ar2 are the same as or different from each other, and may be each independently a phenyl group; a biphenyl group unsubstituted or substituted with a phenyl group; a naphthyl group; a dibenzofuran group; a dibenzothiophene group; a dimethylfluorenyl group; a spirobifluorenyl group; a terphenyl group; a carbazole group unsubstituted or substituted with a phenyl group; -NR201R202 or spiro[fluorene-9,9'-xanthene].

In one embodiment of the present application, R201 and R202 are the same as or different from each other, and may be each independently a C6 to C60 aryl group; or a C2 to C60 heteroaryl group.

In another embodiment, R201 and R202 are the same as or different from each other, and may be each independently a C6 to C40 aryl group; or a C2 to C40 heteroaryl group.

In another embodiment, R201 and R202 are the same as or different from each other, and may be each independently a C6 to C20 aryl group; or a C2 to C20 heteroaryl group.

In another embodiment, R201 and R202 are the same as or different from each other, and may be each independently a phenyl group; a biphenyl group; or a naphthyl group.

In one embodiment of the present application, 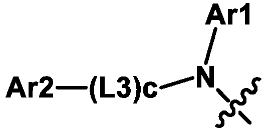 of Chemical Formula 1 may be represented by any one of the following Chemical Formulae 1-1-1 to 1-1-5.

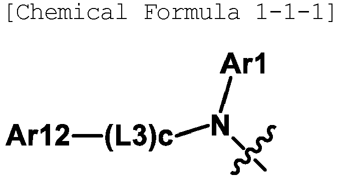

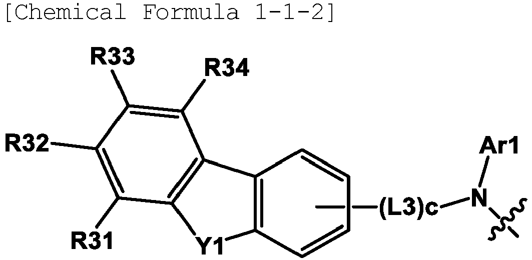

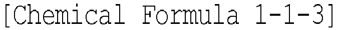

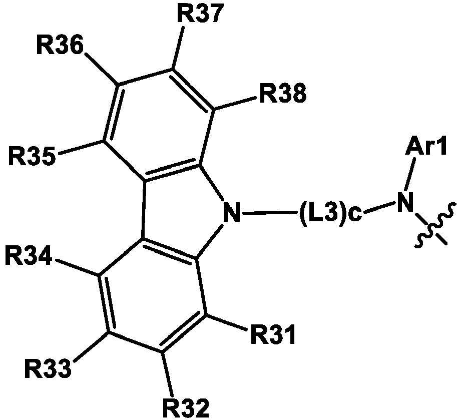

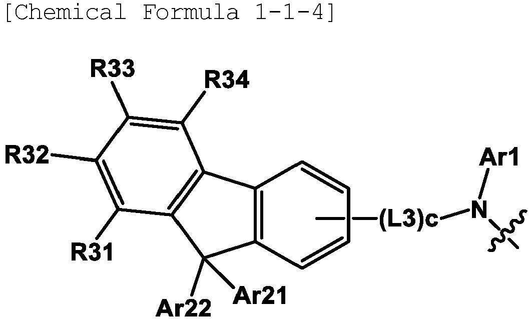

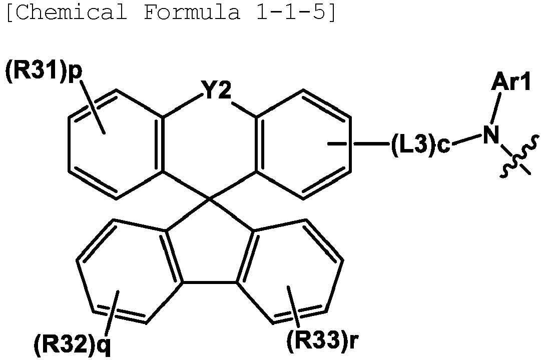

In Chemical Formulae 1-1-1 to 1-1-5,
Ar1, L3 and c have the same definitions as in Chemical Formula 1, and 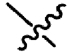 means a position linked to Chemical Formula 1,
Ar12 is a substituted or unsubstituted C6 to C60 aryl group; or -NR201R202,
Y1 is O; S; or NRb,
Y2 is O; or S,
Ar21 and Ar22 are the same as or different from each other, and each independently a substituted or unsubstituted C1 to C60 alkyl group; or a substituted or unsubstituted C6 to C60 aryl group, or two groups adjacent to each other bond to each other to form a substituted or unsubstituted C6 to C60 aromatic hydrocarbon ring,
R31 to R38 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group,
R201 and R202 have the same definitions as in Chemical Formula 1,
Rb is a substituted or unsubstituted C1 to C60 alkyl group; a substituted or unsubstituted C6 to C60 aryl group; or a substituted or unsubstituted C2 to C60 heteroaryl group, and
p, q and r are an integer of 0 to 4.

In one embodiment of the present application, Ar12 may be a substituted or unsubstituted C6 to C60 aryl group; or - NR201R202.

In another embodiment, Ar12 may be a substituted or unsubstituted C6 to C40 aryl group; or -NR201R202.

In another embodiment, Ar12 may be a C6 to C40 aryl group unsubstituted or substituted with a C6 to C10 aryl group; or - NR201R202.

In another embodiment, Ar12 may be a phenyl group; a naphthyl group; a biphenyl group unsubstituted or substituted with a phenyl group; a terphenyl group; a phenanthrenyl group; or -NR201R202.

In one embodiment of the present application, Rb may be a substituted or unsubstituted C1 to C60 alkyl group; a substituted or unsubstituted C6 to C60 aryl group; or a substituted or unsubstituted C2 to C60 heteroaryl group.

In another embodiment, Rb may be a substituted or unsubstituted C6 to C60 aryl group.

In another embodiment, Rb may be a substituted or unsubstituted C6 to C40 aryl group.

In another embodiment, Rb may be a C6 to C40 aryl group.

In another embodiment, Rb may be a monocyclic or polycyclic C6 to C40 aryl group.

In another embodiment, Rb may be a phenyl group.

In one embodiment of the present application, R31 to R38 are the same as or different from each other, and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group.

In another embodiment, R31 to R38 may be hydrogen.

In one embodiment of the present application, Ar21 and Ar22 are the same as or different from each other, and each independently a substituted or unsubstituted C1 to C60 alkyl group; or a substituted or unsubstituted C6 to C60 aryl group, or two groups adjacent to each other may bond to each other to form a substituted or unsubstituted C6 to C60 aromatic hydrocarbon ring.

In another embodiment, Ar21 and Ar22 are the same as or different from each other, and each independently a substituted or unsubstituted C1 to C40 alkyl group; or a substituted or unsubstituted C6 to C40 aryl group, or two groups adjacent to each other may bond to each other to form a substituted or unsubstituted C6 to C40 aromatic hydrocarbon ring.

In another embodiment, Ar21 and Ar22 are the same as or different from each other, and each independently a C1 to C40 alkyl group; or a C6 to C40 aryl group, or two groups adjacent to each other may bond to each other to form a C6 to C40 aromatic hydrocarbon ring.

In another embodiment, Ar21 and Ar22 are the same as or different from each other, and each independently a methyl group; or a phenyl group, or two groups adjacent to each other may bond to each other to form a fluorenyl ring.

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C6 to C60 aryl group.

In another embodiment, R, R' and R" are the same as or different from each other, and may be each independently a substituted or unsubstituted C6 to C60 monocyclic or polycyclic aryl group.

In another embodiment, R, R' and R" are the same as or different from each other, and may be each independently a substituted or unsubstituted C6 to C40 monocyclic aryl group.

In another embodiment, R, R' and R" are the same as or different from each other, and may be each independently a C6 to C20 monocyclic aryl group.

In another embodiment, R, R' and R" may be a phenyl group.

According to one embodiment of the present application, Chemical Formula 1 may be represented by any one of the following compounds, but is not limited thereto.

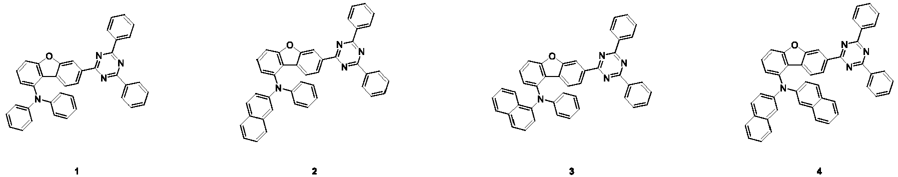

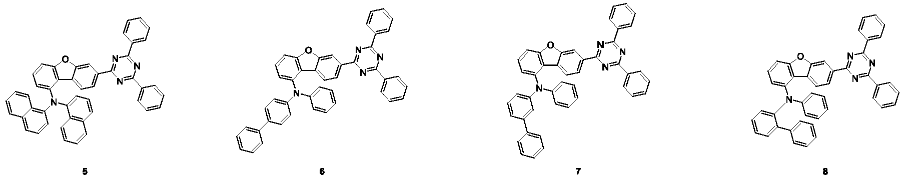

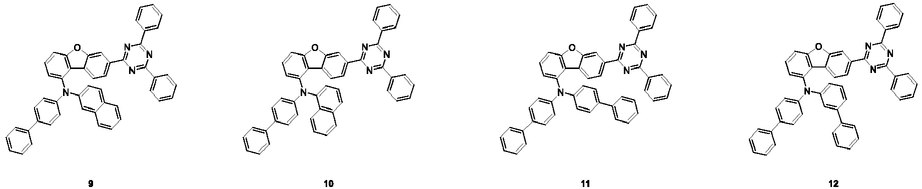

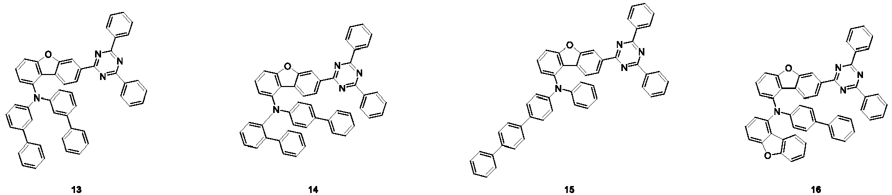

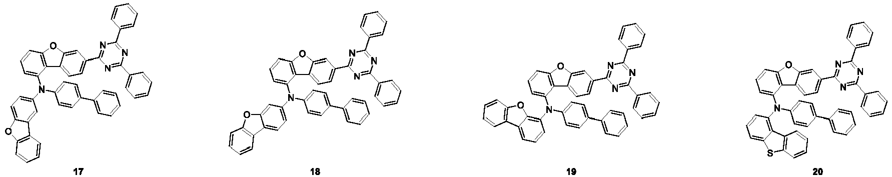

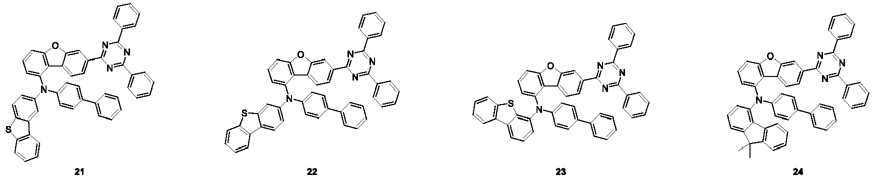

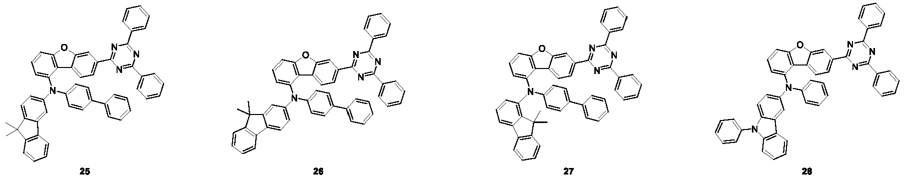

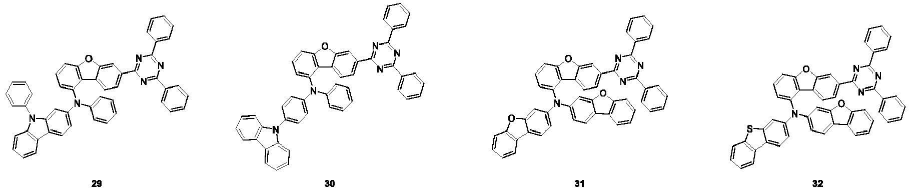

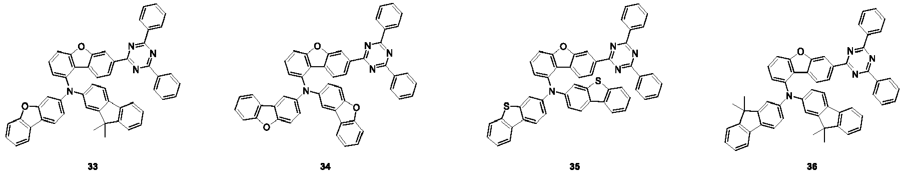

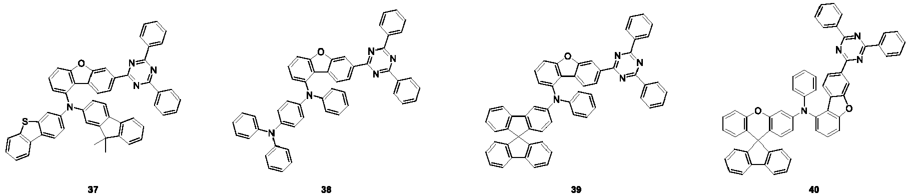

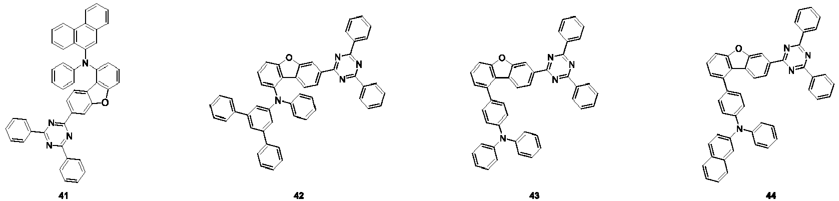

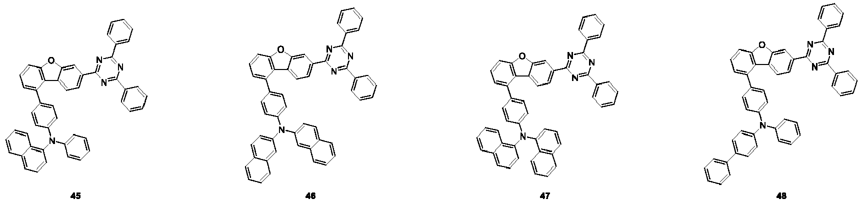

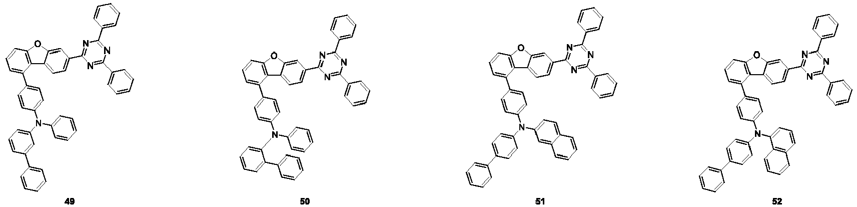

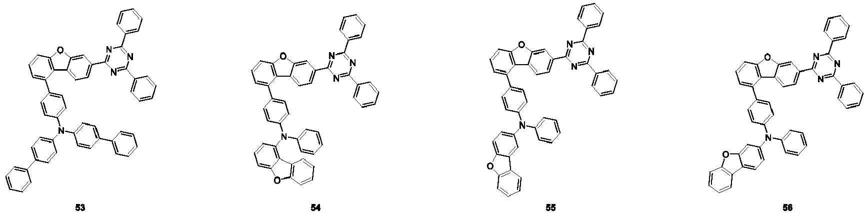

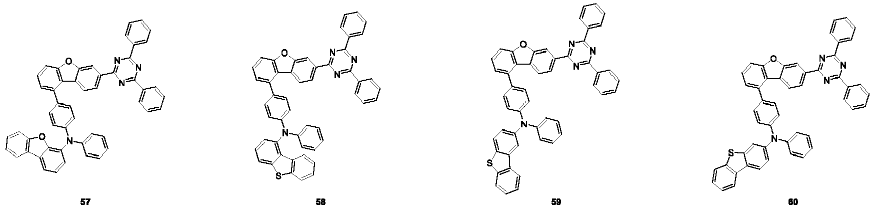

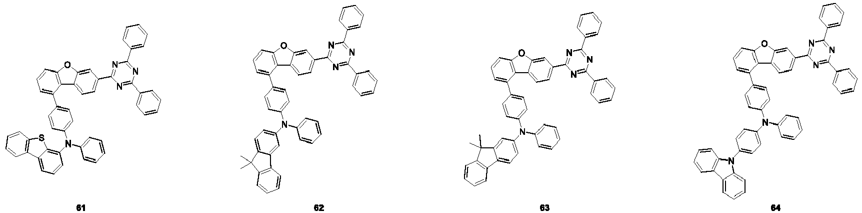

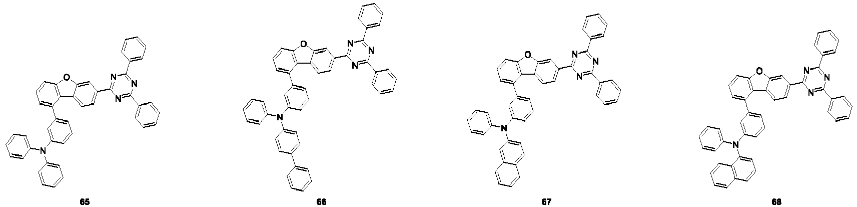

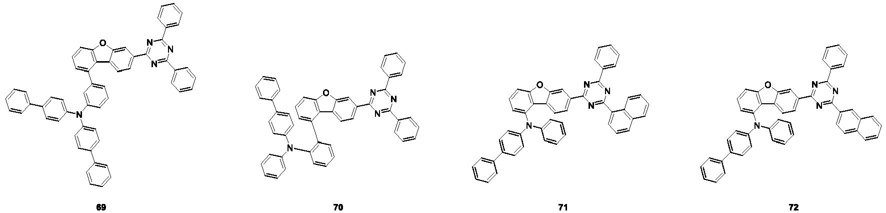

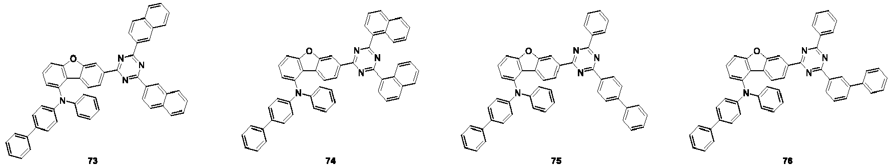

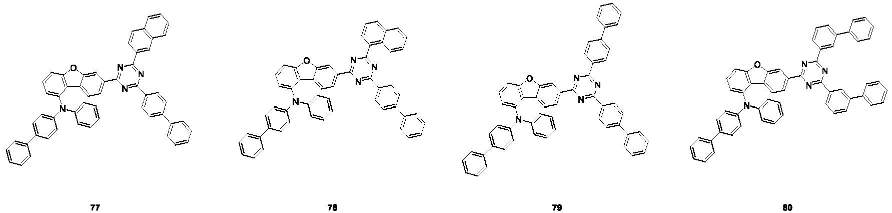

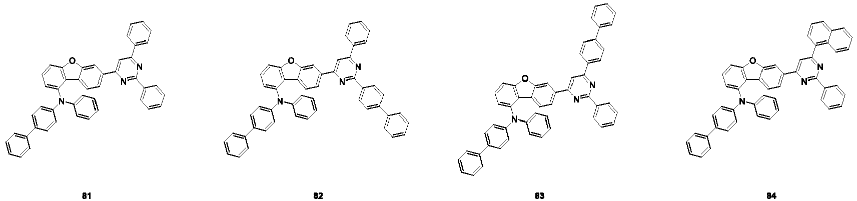

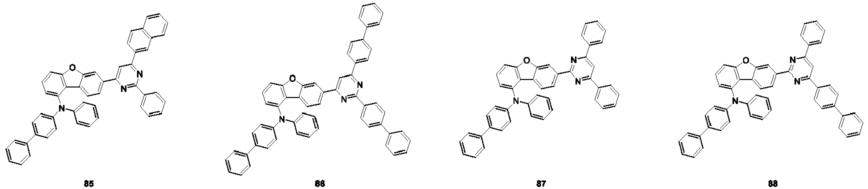

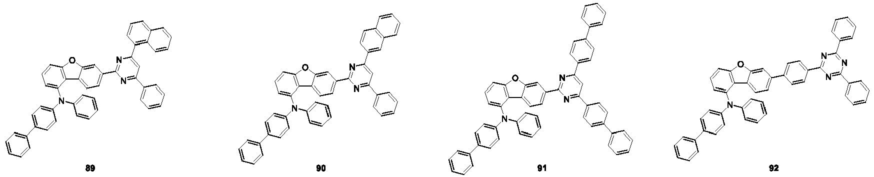

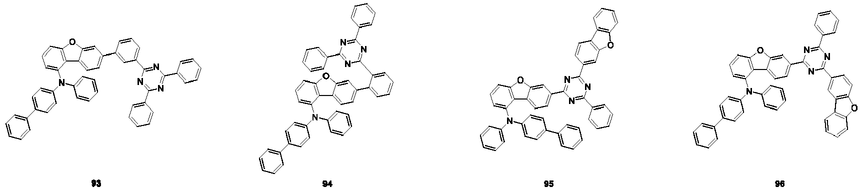

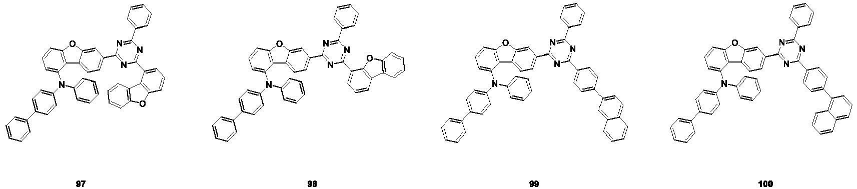

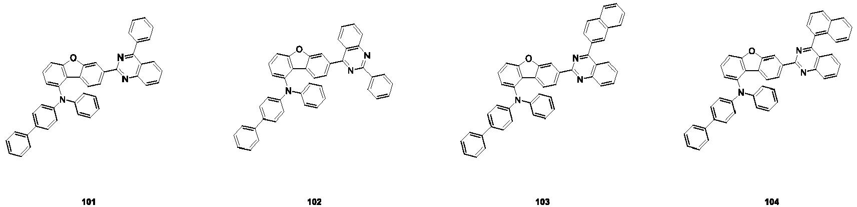

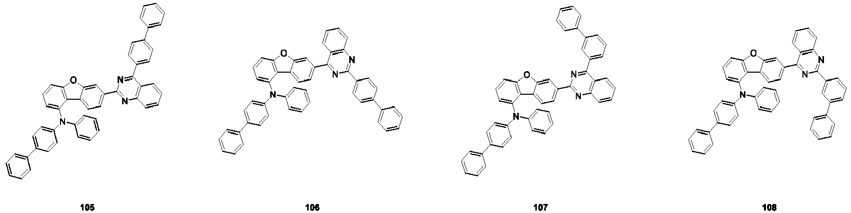

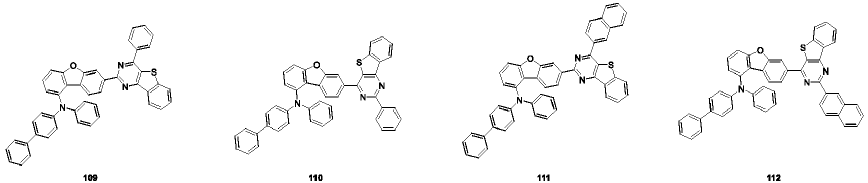

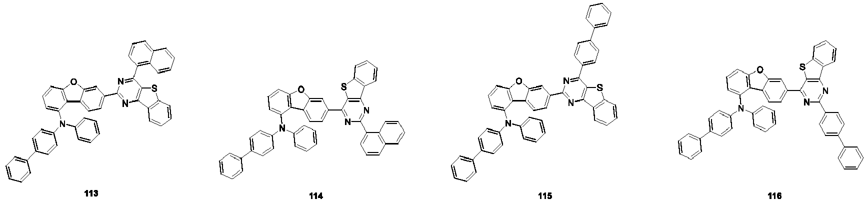

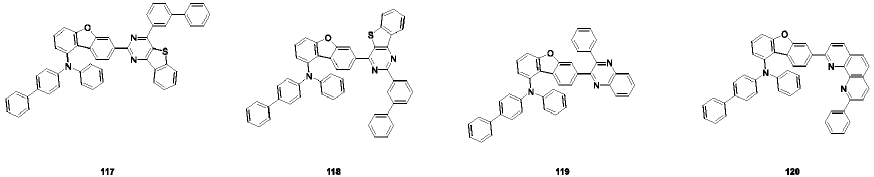

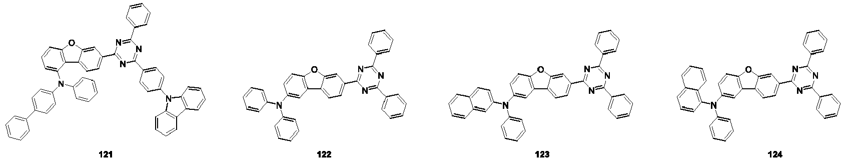

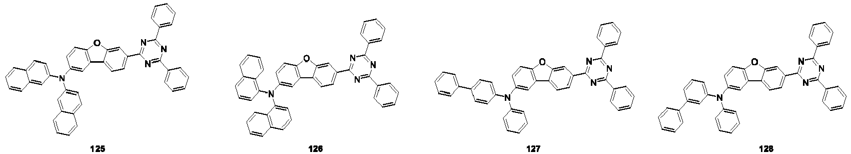

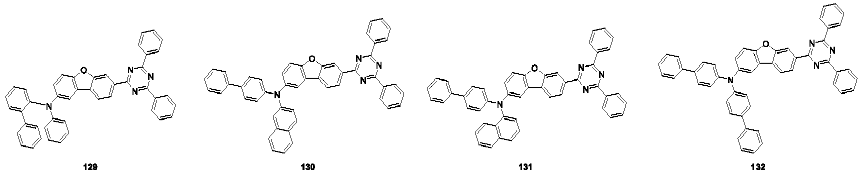

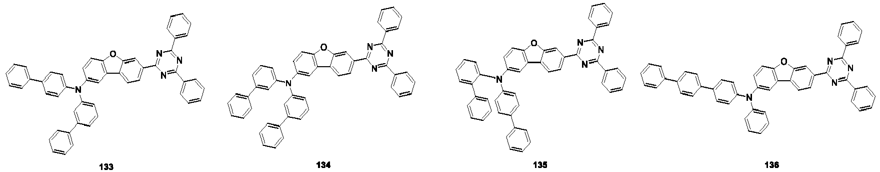

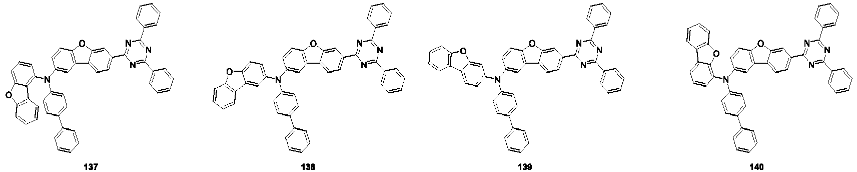

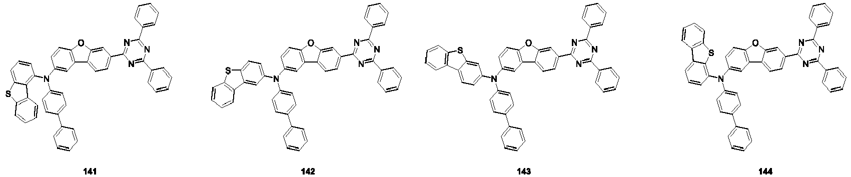

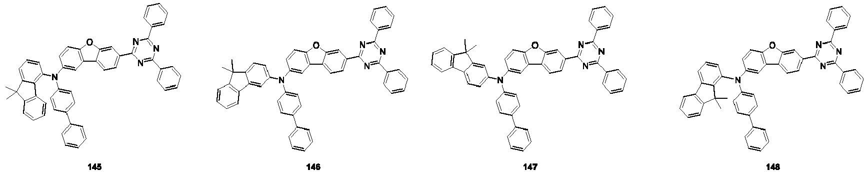

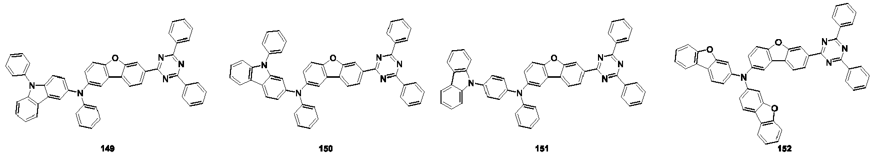

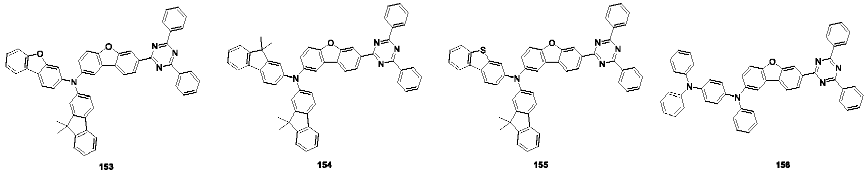

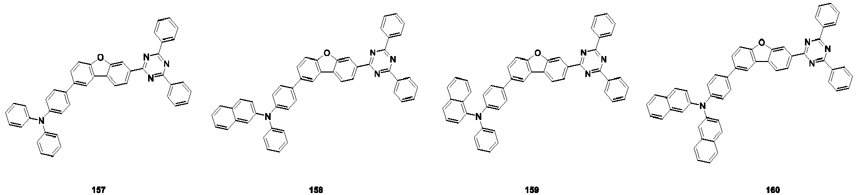

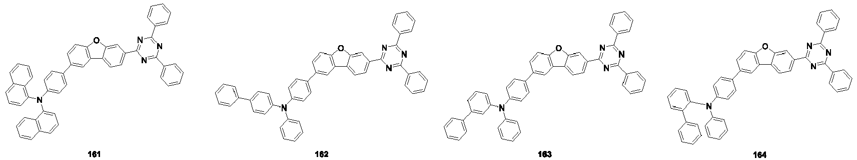

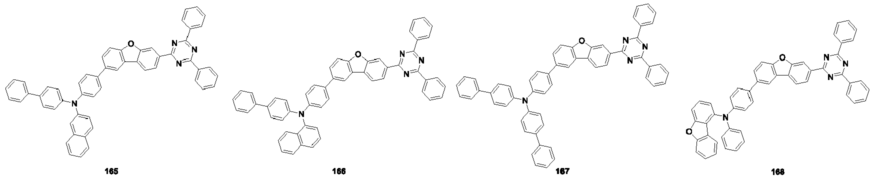

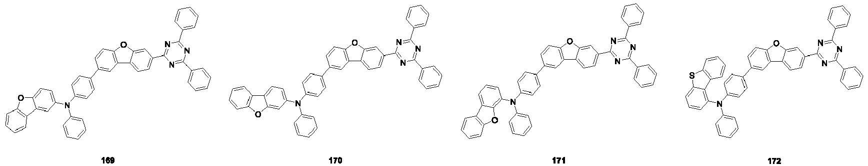

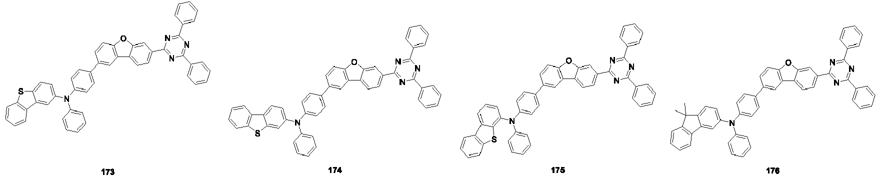

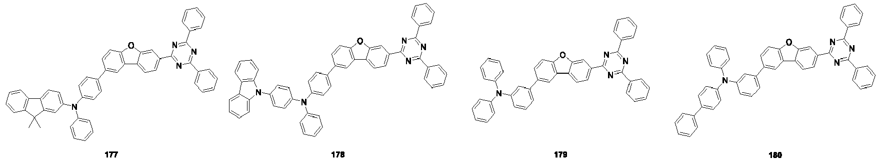

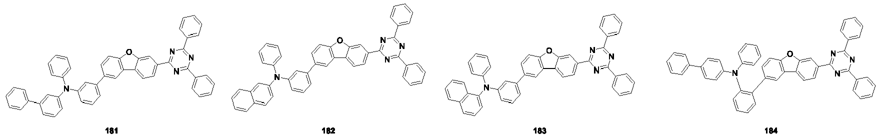

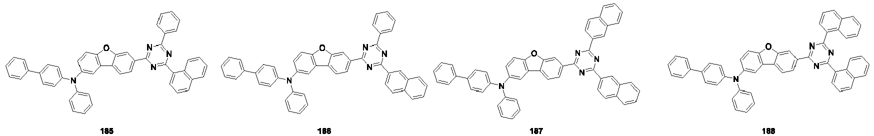

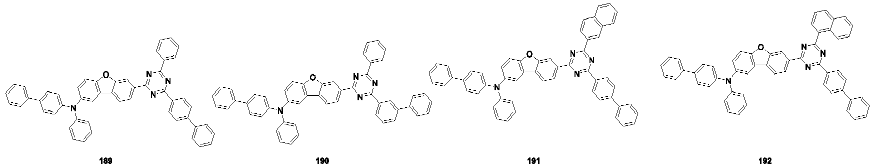

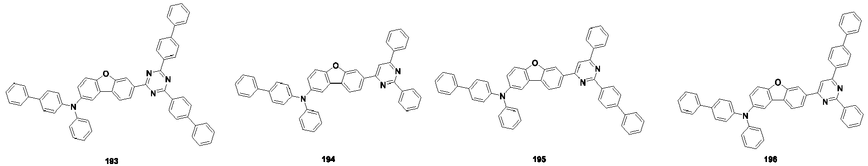

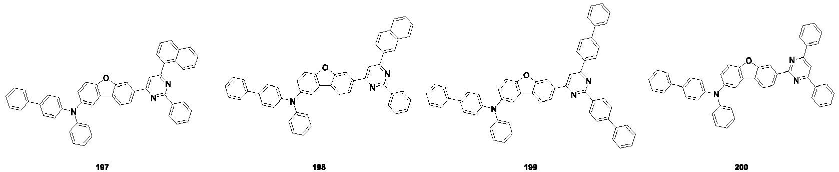

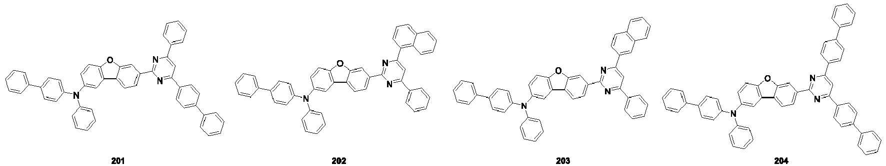

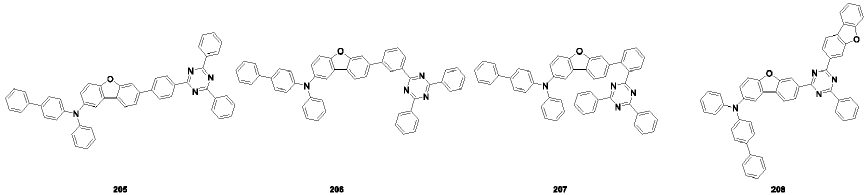

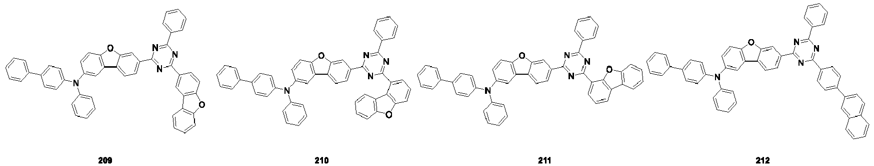

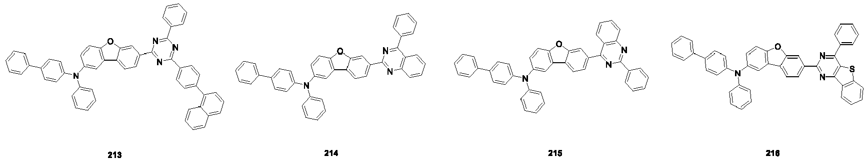

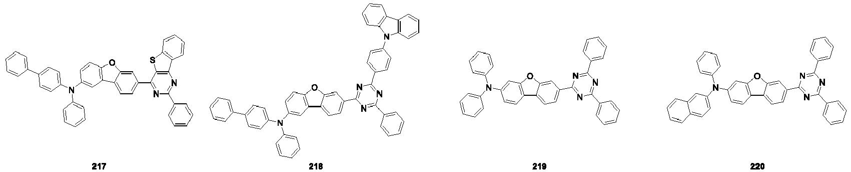

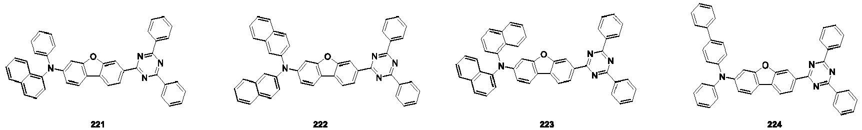

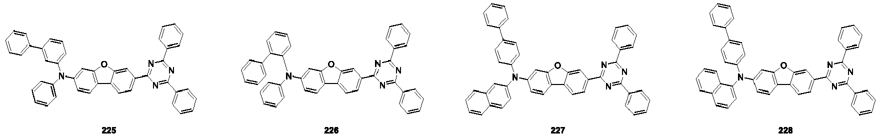

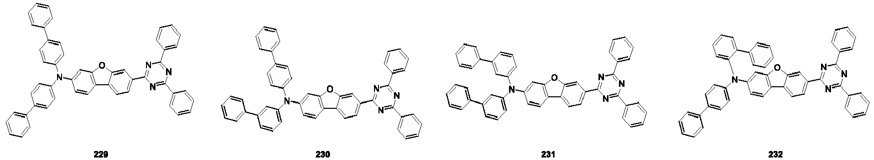

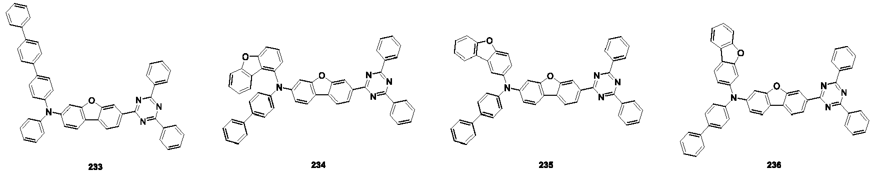

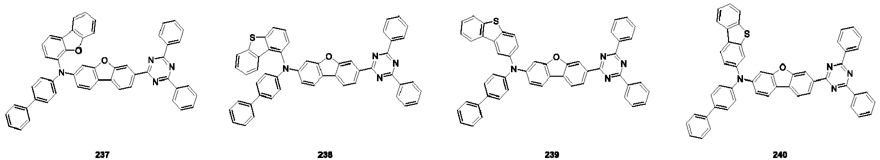

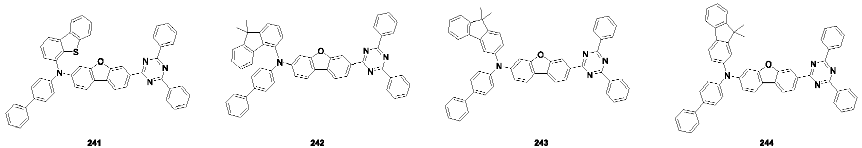

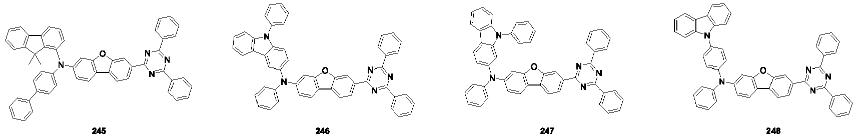

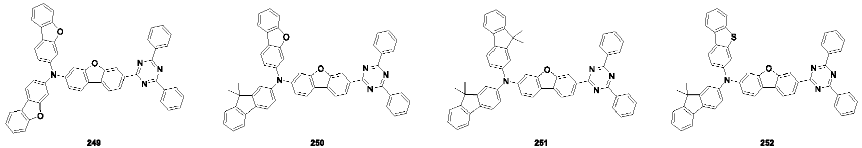

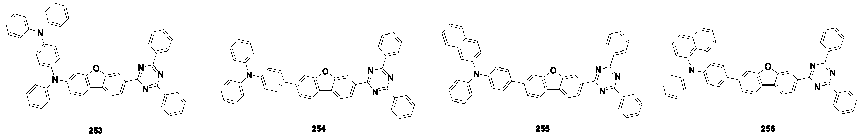

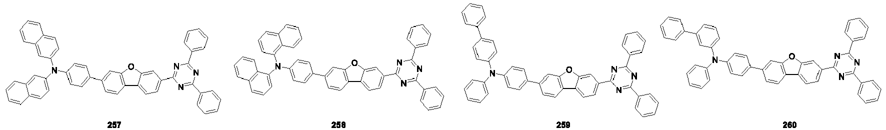

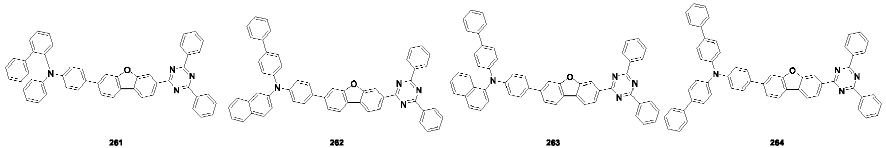

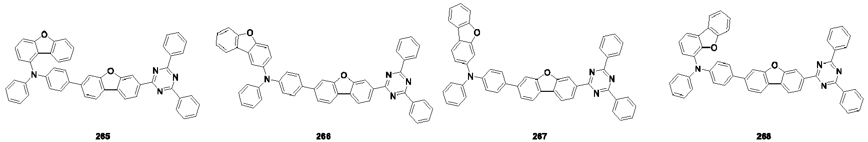

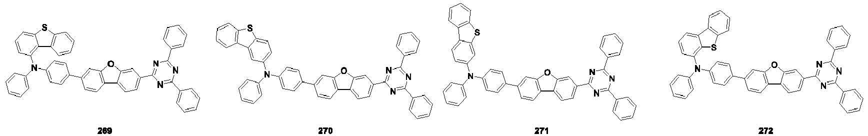

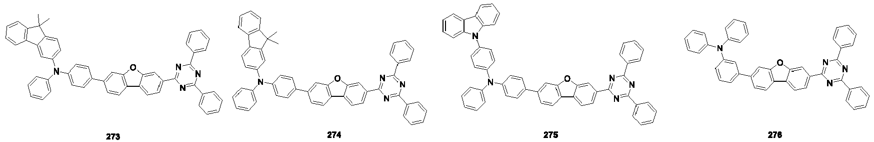

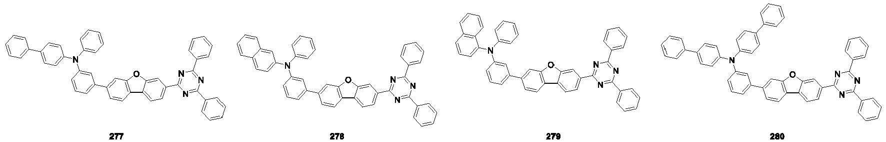

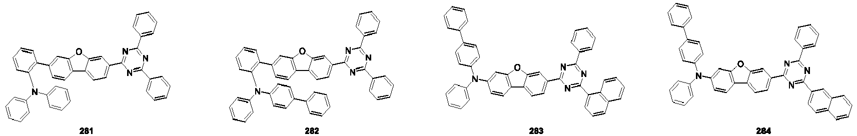

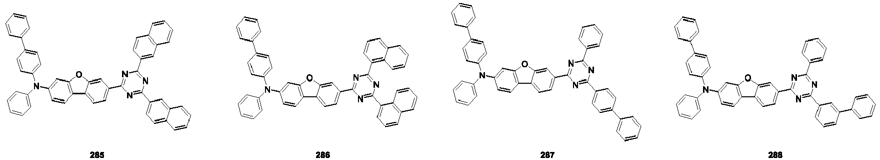

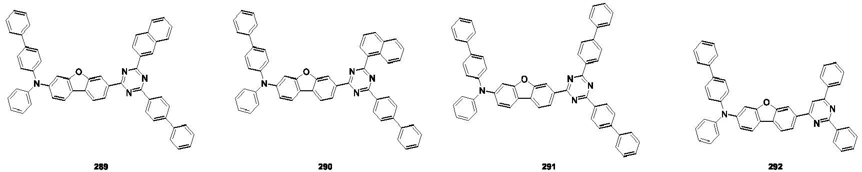

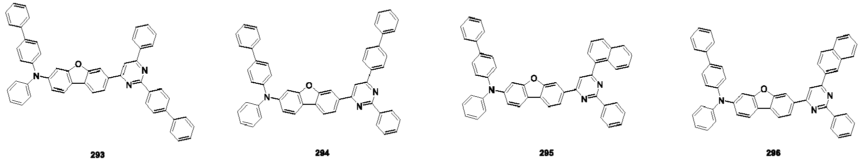

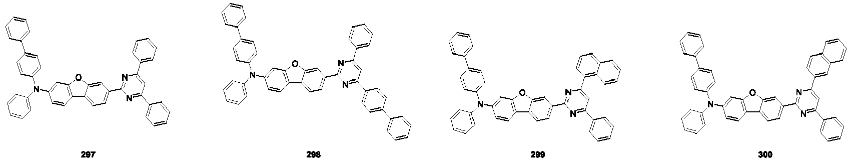

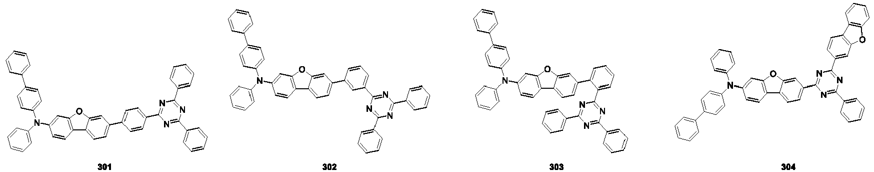

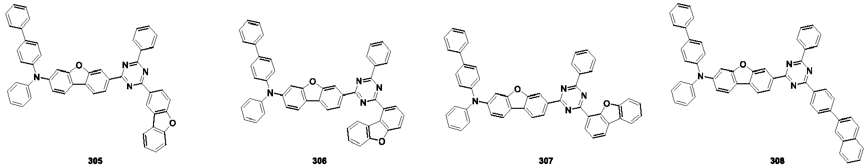

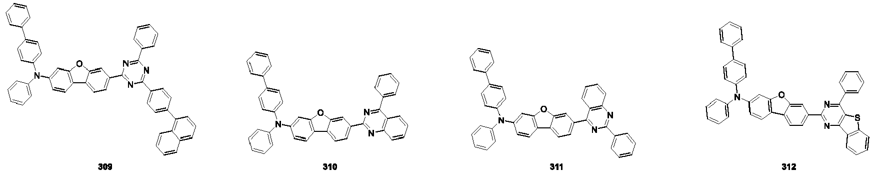

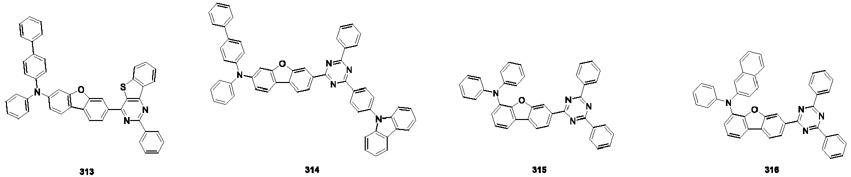

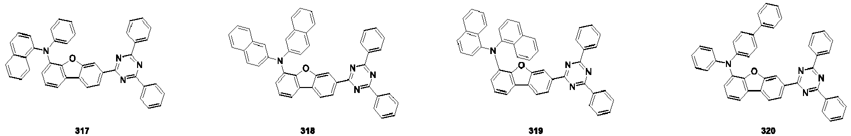

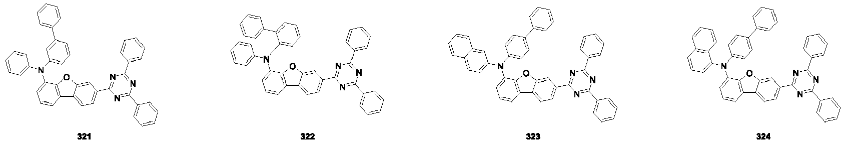

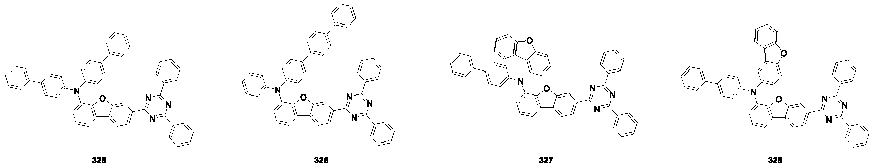

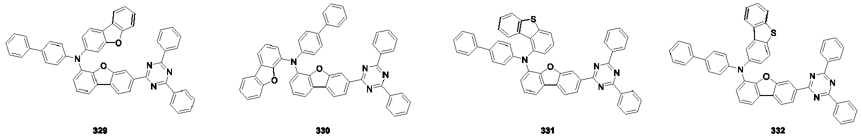

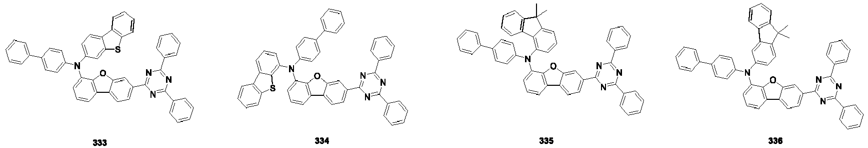

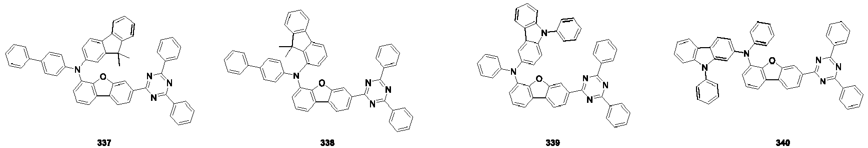

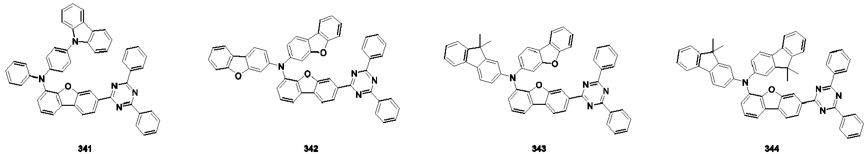

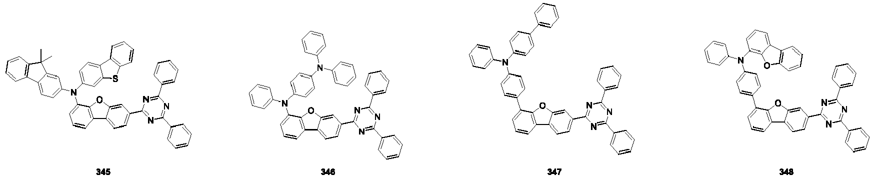

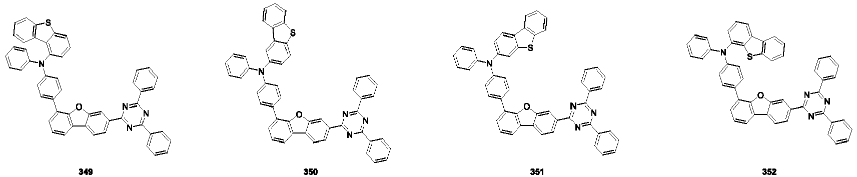

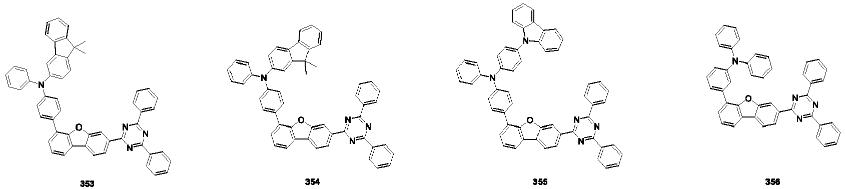

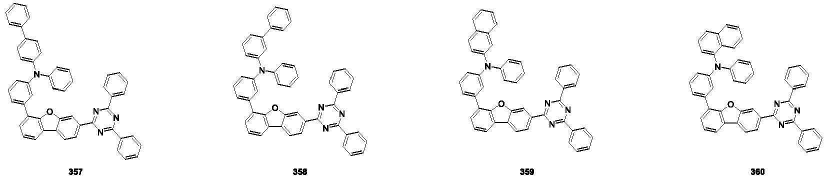

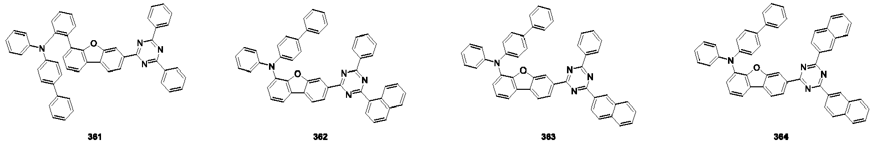

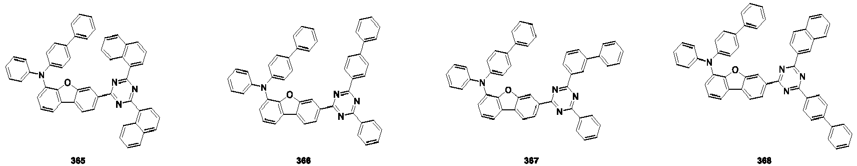

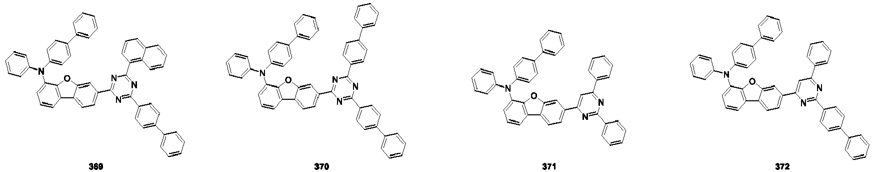

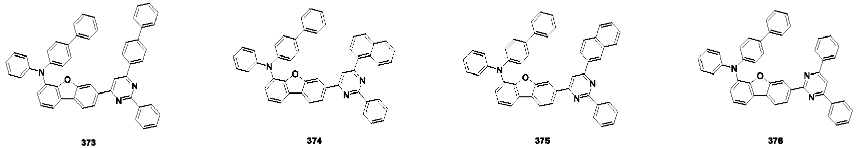

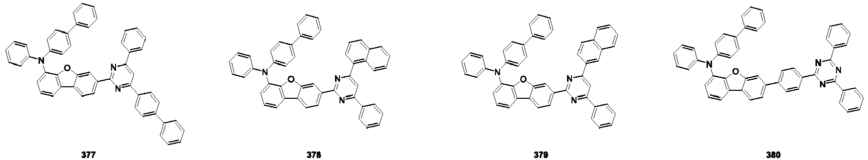

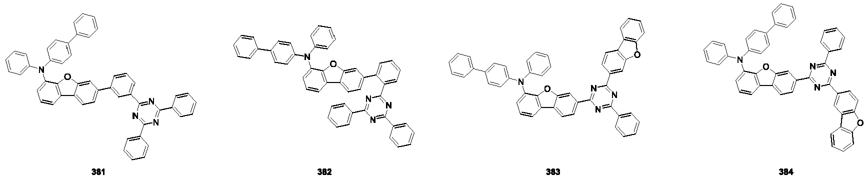

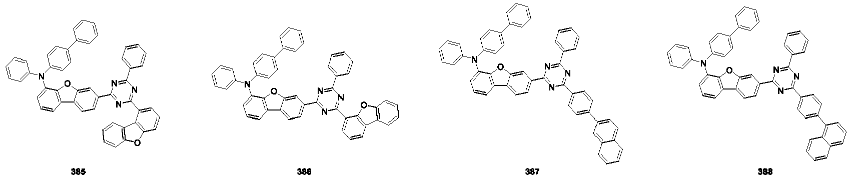

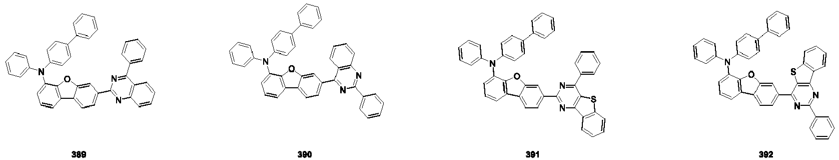

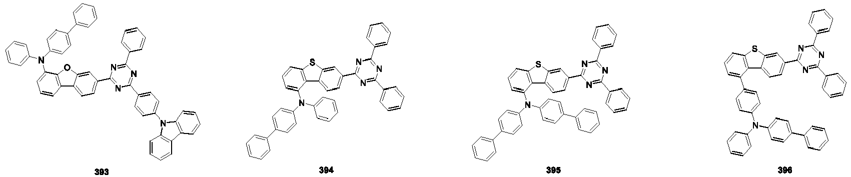

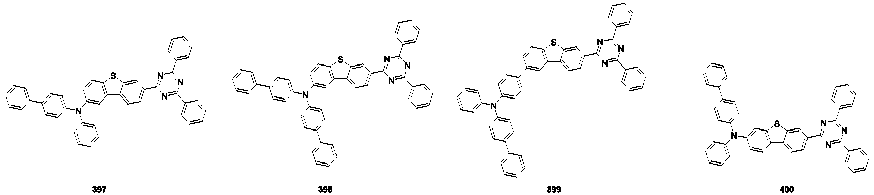

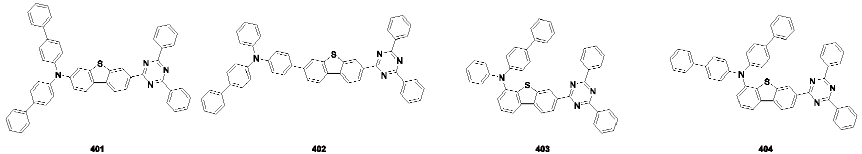

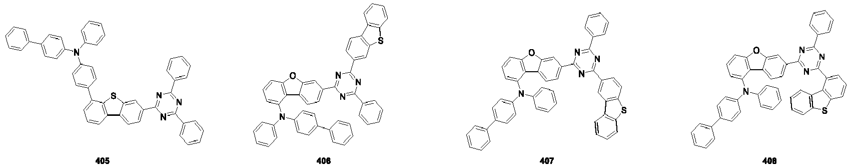

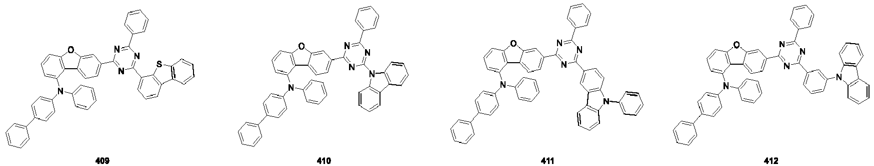

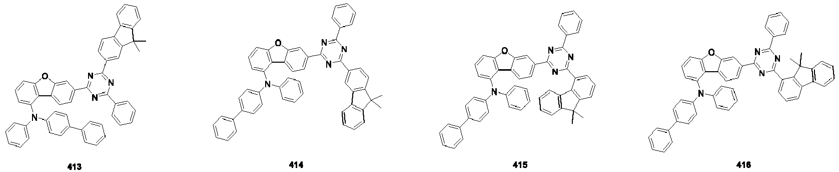

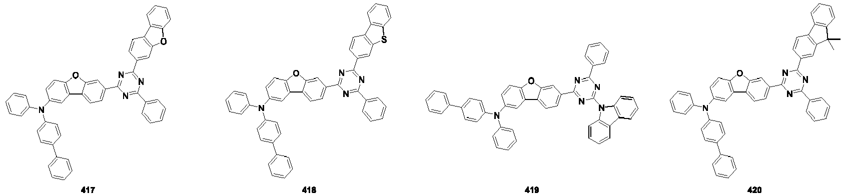

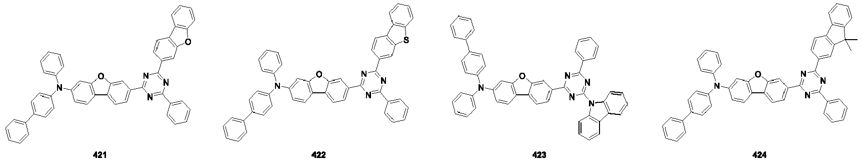

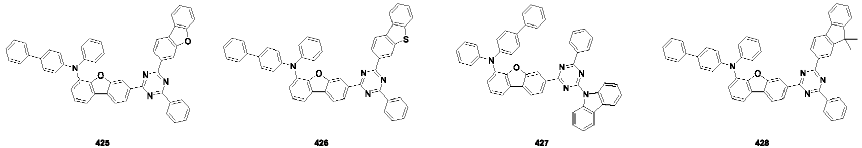

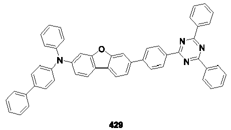

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise one or more types of the heterocyclic compound according to Chemical Formula 1.

Another embodiment provides an organic light emitting device comprising a first electrode; a second electrode provided opposite to the first electrode; and one or more organic material layers provided between the first electrode and the second electrode, wherein one or more layers of the organic material layers comprise one type of the heterocyclic compound according to Chemical Formula 1.

Another embodiment provides an organic light emitting device comprising a first electrode; a second electrode provided opposite to the first electrode; and one or more organic material layers provided between the first electrode and the second electrode, wherein one or more layers of the organic material layers comprise two types of the heterocyclic compound according to Chemical Formula 1.

Specific details on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of a blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of a green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of a red organic light emitting device.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

In another embodiment, the organic material layer comprising the heterocyclic compound comprises the heterocyclic compound represented by Chemical Formula 1 as a host, and an iridium-based dopant may be used therewith.

In the organic light emitting device of the present disclosure, the organic material layer comprises an electron injection layer or an electron transfer layer, and the electron transfer layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

In the organic light emitting device of the present disclosure, the organic material layer comprises a hole transfer layer, and the hole transfer layer may comprise the heterocyclic compound.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

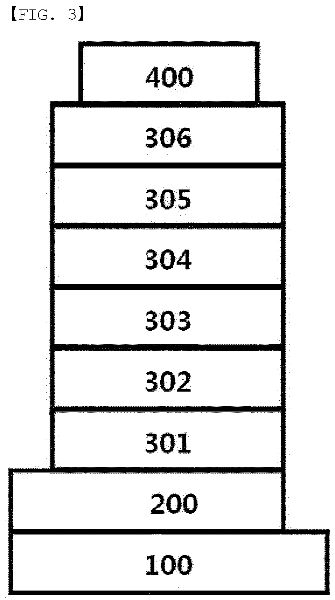

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the compound of Chemical Formula 1 may further comprise other materials as necessary.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 6

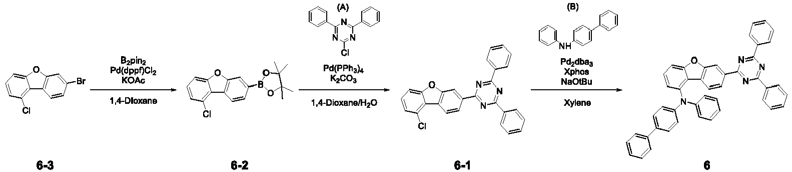

### Preparation of Intermediate 6-2

7-Bromo-1-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 6-2 (16.8 g, 72%).

### Preparation of Intermediate 6-1

Intermediate 6-2 (16.8 g, 51.1 mmol), 2-chloro-4,6-diphenyl-1,3,5-triazine (13.7 g, 51.1 mmol), Pd(PPh₃)₄ (2.95 g, 2.56 mmol) and K₂CO₃ (21.2 g, 153 mmol) were introduced to 1,4-dioxane/H₂O (170 mL/50 mL), and stirred for 2 hours at 100°C. The result was cooled to room temperature, filtered, and then washed with H₂O and MeOH to obtain Intermediate 6-1 (20.2 g, 91%) .

### Preparation of Compound 6

Intermediate 6-1 (7.00 g, 16.1 mmol), N-phenyl-[1,1'-biphenyl]-4-amine (4.35 g, 17.7 mmol), Pd₂dba₃ (0.74 g, 0.81 mmol), Xphos (0.77 g, 1.61 mmol) and t-BuONa (3.09 g, 32.0 mmol) were introduced to xylene, and stirred for 2 hours at 150°C. The result was cooled to room temperature and left unattended for 12 hours, and produced solids were filtered. The result was silica gel filtered and then vacuum concentrated, and produced solids were filtered to obtain Compound 6 (8.76 g, 85%).

The following compounds were synthesized in the same manner as in Preparation of Compound 6 of Preparation Example 1 except that A and B of the following Table 1 were used as the intermediates.

**[Table 1]**

| Compound | A | B | Yield |
|---|---|---|---|
| 11 | 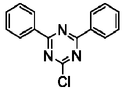 | 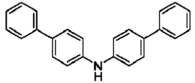 | 72% |
| 14 | 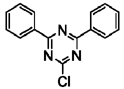 | 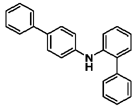 | 74% |
| 18 | 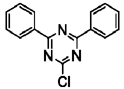 | 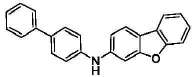 | 68% |
| 26 | 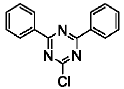 | 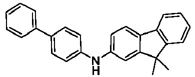 | 80% |
| 30 | 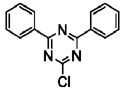 | 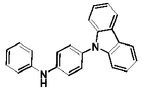 | 78% |
| 38 | 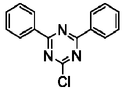 | 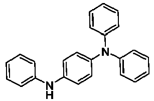 | 81% |
| 72 | 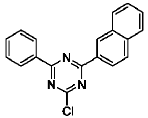 | 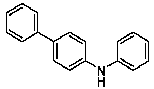 | 65% |
| 73 | 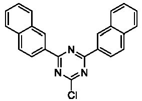 | 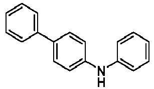 | 71% |
| 75 | 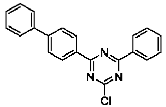 | 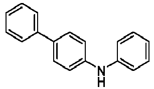 | 65% |
| 76 | 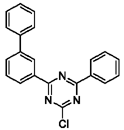 | 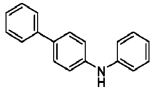 | 71% |
| 81 | 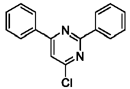 | 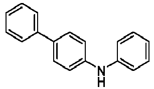 | 64% |
| 87 | 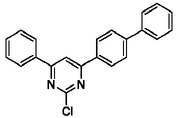 | 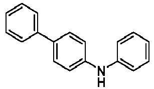 | 66% |
| 92 | 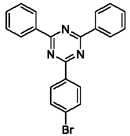 | 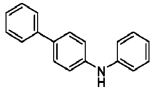 | 73% |
| 93 | 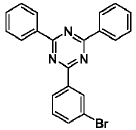 | 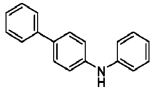 | 69% |
| 98 | 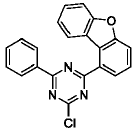 | 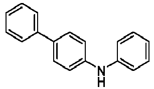 | 70% |
| 99 | 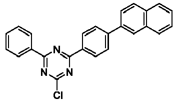 | 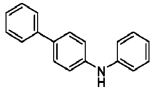 | 80% |
| 101 | 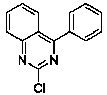 | 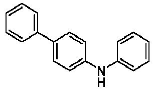 | 76% |
| 109 | 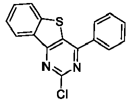 | 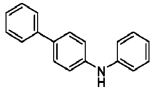 | 74% |
| 115 | 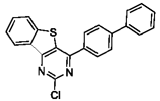 | 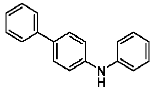 | 72% |

### <Preparation Example 2> Preparation of Compound 47

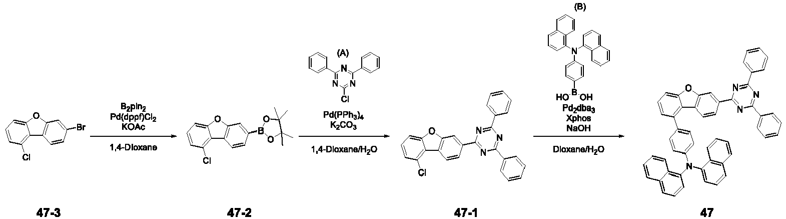

### Preparation of Intermediate 47-2

7-Bromo-1-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 47-2 (16.8 g, 72%).

### Preparation of Intermediate 47-1

Intermediate 47-2 (16.8 g, 72.3 mmol), 2-chloro-4,6-diphenyl-1,3,5-triazine (13.7 g, 51.1 mmol), Pd(PPh₃)₄ (2.95 g, 2.56 mmol) and K₂CO₃ (21.2 g, 153 mmol) were introduced to 1,4-dioxane/H₂O (170 mL/50 mL), and stirred for 2 hours at 100°C. The result was cooled to room temperature, filtered, and then washed with H₂O and MeOH to obtain Intermediate 47-1 (20.2 g, 91%) .

### Preparation of Compound 47

Intermediate 47-1 (7.00 g, 16.1 mmol), (4-(di(naphthalen-1-yl)amino)phenyl)boronic acid (6.89 g, 17.7 mmol), Pd₂dba₃ (0.74 g, 0.81 mmol), Xphos (0.77 g, 1.61 mmol) and NaOH (1.28 g, 32.0 mmol) were introduced to dioxane/H₂O (70 mL/20 mL), and stirred for 2 hours at 150°C. The result was cooled to room temperature and left unattended for 12 hours, and produced solids were filtered. The result was silica gel filtered and then vacuum concentrated, and produced solids were filtered to obtain Compound 47 (8.85g, 74%).

The following compounds were synthesized in the same manner as in Preparation of Compound 47 of Preparation Example 2 except that A and B of the following Table 2 were used as the intermediates.

**[Table 2]**

| Compound | A | B | Yield |
|---|---|---|---|
| 48 | 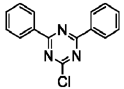 | 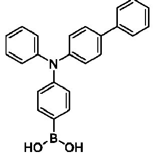 | 69% |
| 52 | 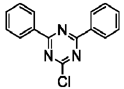 | 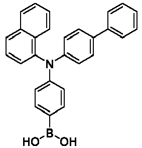 | 77% |
| 53 | 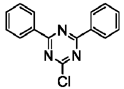 | 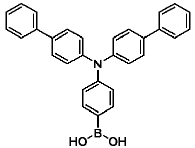 | 72% |
| 57 | 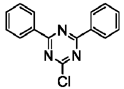 | 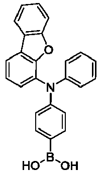 | 65% |

### <Preparation Example 3> Preparation of Compound 127

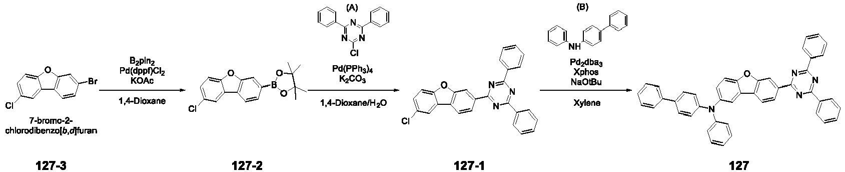

### Preparation of Intermediate 127-2

7-Bromo-2-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 127-2 (15.9 g, 68%) .

### Preparation of Intermediate 127-1

Intermediate 127-2 (15.9 g, 48.4 mmol), 2-chloro-4,6-diphenyl-1,3,5-triazine (13.0 g, 48.4 mmol), Pd(PPh₃)₄ (2.80 g, 2.42 mmol) and K₂CO₃ (20.0 g, 145 mmol) were introduced to 1,4-dioxane/H₂O (160 mL/50 mL), and stirred for 2 hours at 100°C. The result was cooled to room temperature, filtered, and then washed with H₂O and MeOH to obtain Intermediate 127-1 (19.8 g, 94%) .

### Preparation of Compound 127

Intermediate 127-1 (7.00 g, 16.1 mmol), N-phenyl-[1,1'-biphenyl]-4-amine (4.35 g, 17.7 mmol), Pd₂dba₃ (0.74 g, 0.81 mmol), Xphos (0.77 g, 1.61 mmol) and t-BuONa (3.09 g, 32.0 mmol) were introduced to xylene, and stirred for 2 hours at 150°C. The result was cooled to room temperature and left unattended for 12 hours, and produced solids were filtered. The result was silica gel filtered and then vacuum concentrated, and produced solids were filtered to obtain Compound 127 (6.79 g, 66%).

The following compounds were synthesized in the same manner as in Preparation of Compound 127 of Preparation Example 3 except that A and B of the following Table 3 were used as the intermediates.

**[Table 3]**

| Compound | A | B | Yield |
|---|---|---|---|
| 132 | 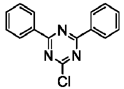 | 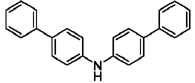 | 71% |
| 156 | 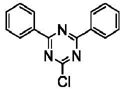 | 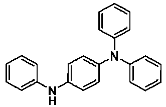 | 66% |
| 186 | 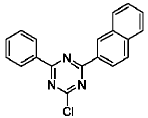 | 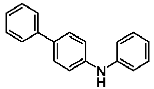 | 74% |

### <Preparation Example 4> Preparation of Compound 162

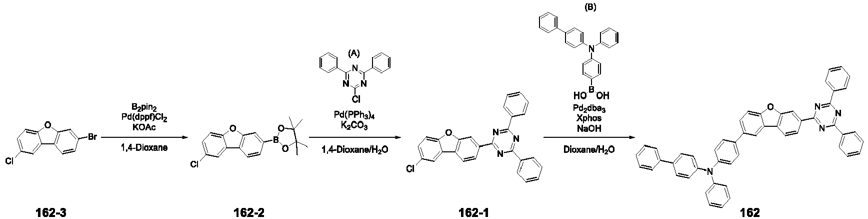

### Preparation of Intermediate 162-2

7-Bromo-2-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 162-2 (15.9 g, 68%) .

### Preparation of Intermediate 162-1

Intermediate 162-2 (16.8 g, 72.3 mmol), 2-chloro-4,6-diphenyl-1,3,5-triazine (13.7 g, 51.1 mmol), Pd(PPh₃)₄ (2.95 g, 2.56 mmol) and K₂CO₃ (21.2 g, 153 mmol) were introduced to 1,4-dioxane/H₂O (170 mL/50 mL), and stirred for 2 hours at 100°C. The result was cooled to room temperature, filtered, and then washed with H₂O and MeOH to obtain Intermediate 162-1 (19.8 g, 94%) .

### Preparation of Compound 162

Intermediate 162-1 (7.00 g, 16.1 mmol), (4-([1,1'-biphenyl]-4-yl(phenyl)amino)phenyl)boronic acid (6.46 g, 17.7 mmol), Pd₂dba₃ (0.74 g, 0.81 mmol), Xphos (0.77 g, 1.61 mmol) and NaOH (1.28 g, 32.0 mmol) were introduced to dioxane/H₂O (70 mL/ 20 mL), and stirred for 2 hours at 150°C. The result was cooled to room temperature and left unattended for 12 hours, and produced solids were filtered. The result was silica gel filtered and then vacuum concentrated, and produced solids were filtered to obtain Compound 162 (7.95 g, 68%).

The following compounds were synthesized in the same manner as in Preparation of Compound 162 of Preparation Example 4 except that A and B of the following Table 4 were used as the intermediates.

**[Table 4]**

| Compound | A | B | Yield |
|---|---|---|---|
| 183 | 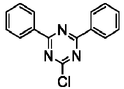 | 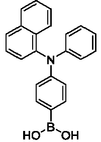 | 65% |

### <Preparation Example 5> Preparation of Compound 229

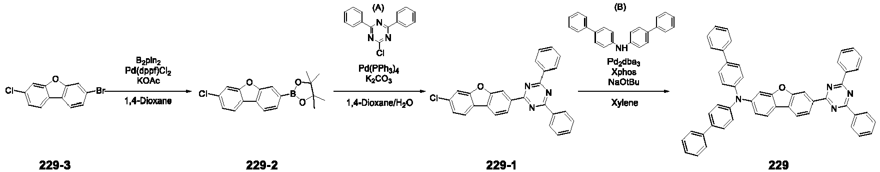

### Preparation of Intermediate 229-2

7-Bromo-3-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 229-2 (15.9 g, 68%) .

### Preparation of Intermediate 229-1

Intermediate 229-2 (15.9 g, 48.4 mmol), 2-chloro-4,6-diphenyl-1,3,5-triazine (13.0 g, 48.4 mmol), Pd(PPh₃)₄ (2.80 g, 2.42 mmol) and K₂CO₃ (20.0 g, 145 mmol) were introduced to 1,4-dioxane/H₂O (160 mL/50 mL), and stirred for 2 hours at 100°C. The result was cooled to room temperature, filtered, and then washed with H₂O and MeOH to obtain Intermediate 229-1 (19.8 g, 94%) .

### Preparation of Compound 229

Intermediate 229-1 (7.00 g, 16.1 mmol), di([1,1'-biphenyl]-4-yl)amine (5.69 g, 17.7 mmol), Pd₂dba₃ (0.74 g, 0.81 mmol), Xphos (0.77 g, 1.61 mmol) and t-BuONa (3.09 g, 32.0 mmol) were introduced to xylene, and stirred for 2 hours at 150°C. The result was cooled to room temperature and left unattended for 12 hours, and produced solids were filtered. The result was silica gel filtered and then vacuum concentrated, and produced solids were filtered to obtain Compound 229 (7.13 g, 61%).

The following compounds were synthesized in the same manner as in Preparation of Compound 229 of Preparation Example 5 except that A and B of the following Table 5 were used as the intermediates.

**[Table 5]**

| Compound | A | B | Yield |
|---|---|---|---|
| 301 | 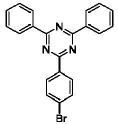 | 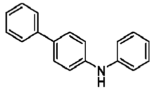 | 71% |
| 304 | 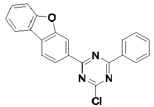 | 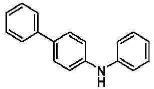 | 65% |
| 308 | 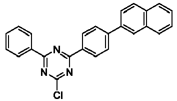 | 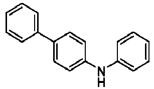 | 69% |

### <Preparation Example 6> Preparation of Compound 259

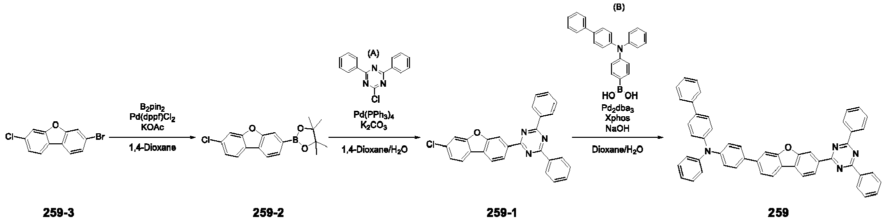

### Preparation of Intermediate 259-2

7-Bromo-3-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 259-2 (15.9 g, 68%) .

### Preparation of Intermediate 259-1

Intermediate 259-2 (16.8 g, 72.3 mmol), 2-chloro-4,6-diphenyl-1,3,5-triazine (13.7 g, 51.1 mmol), Pd(PPh₃)₄ (2.95 g, 2.56 mmol) and K₂CO₃ (21.2 g, 153 mmol) were introduced to 1,4-dioxane/H₂O (170 mL/50 mL), and stirred for 2 hours at 100°C. The result was cooled to room temperature, filtered, and then washed with H₂O and MeOH to obtain Intermediate 259-1 (19.8 g, 94%) .

### Preparation of Compound 259

Intermediate 259-1 (7.00 g, 16.1 mmol), (4-([1,1'-biphenyl]-4-yl(phenyl)amino)phenyl)boronic acid (6.46 g, 17.7 mmol), Pd₂dba₃ (0.74 g, 0.81 mmol), Xphos (0.77 g, 1.61 mmol) and NaOH (1.28 g, 32.0 mmol) were introduced to dioxane/H₂O (70 mL/20 mL), and stirred for 2 hours at 150°C. The result was cooled to room temperature and left unattended for 12 hours, and produced solids were filtered. The result was silica gel filtered and then vacuum concentrated, and produced solids were filtered to obtain Compound 259 (7.95 g, 68%).

### <Preparation Example 7> Preparation of Compound 325

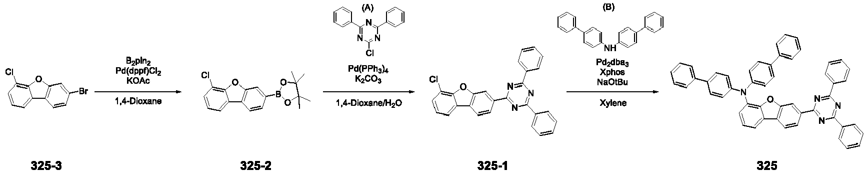

### Preparation of Intermediate 325-2

7-Bromo-4-chlorodibenzo[b,d]furan (20.0 g, 71.0 mmol), B₂pin₂ (27.1 g, 107 mmol), Pd(dppf)Cl₂ (5.20 g, 7.10 mmol) and KOAc (20.9 g, 213 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 325-2 (15.9 g, 68%) .

### Preparation of Intermediate 325-1

Intermediate 325-2 (15.9 g, 48.4 mmol), 2-chloro-4,6-diphenyl-1,3,5-triazine (13.0 g, 48.4 mmol), Pd(PPh₃)₄ (2.80 g, 2.42 mmol) and K₂CO₃ (20.0 g, 145 mmol) were introduced to 1,4-dioxane/H₂O (160 mL/50 mL), and stirred for 2 hours at 100°C. The result was cooled to room temperature, filtered, and then washed with H₂O and MeOH to obtain Intermediate 325-1 (19.8 g, 94%) .

### Preparation of Compound 325

Intermediate 325-1 (7.00 g, 16.1 mmol), di([1,1'-biphenyl]-4-yl)amine (5.69 g, 17.7 mmol), Pd₂dba₃ (0.74 g, 0.81 mmol), Xphos (0.77 g, 1.61 mmol) and t-BuONa (3.09 g, 32.0 mmol) were introduced to xylene, and stirred for 2 hours at 150°C. The result was cooled to room temperature and left unattended for 12 hours, and produced solids were filtered. The result was silica gel filtered and then vacuum concentrated, and produced solids were filtered to obtain Compound 325 (7.13 g, 61%).

The following compounds were synthesized in the same manner as in Preparation of Compound 325 of Preparation Example 7 except that A and B of the following Table 6 were used as the intermediates.

**[Table 6]**

| Compound | A | B | Yield |
|---|---|---|---|
| 332 | 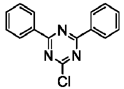 | 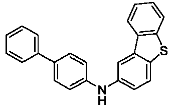 | 68% |
| 337 | 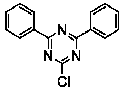 | 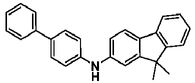 | 76% |

### <Preparation Example 9> Preparation of Compound 395

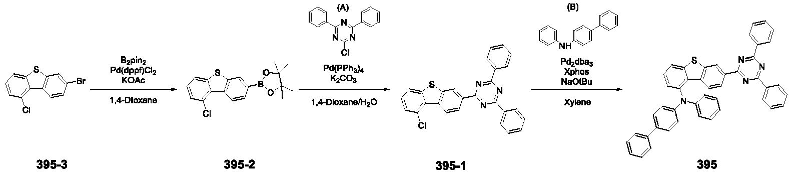

### Preparation of Intermediate 395-2

7-Bromo-1-chlorodibenzo[b,d]thiophene (20.0 g, 67.2 mmol), B₂pin₂ (25.6 g, 101 mmol), Pd(dppf)Cl₂ (4.92 g, 6.72 mmol) and KOAc (19.8 g, 202 mmol) were introduced to 1,4-dioxane (200 mL), and stirred for 3 hours at 100°C. The result was filtered while hot, and washed several times with DCM. The filtrate was vacuum concentrated, and then silica gel filtered. The result was washed with MeOH to obtain Intermediate 395-2 (19.1 g, 82%).

### Preparation of Intermediate 395-1

Intermediate 395-2 (19.0 g, 55.1 mmol), 2-chloro-4,6-diphenyl-1,3,5-triazine (14.8 g, 55.1 mmol), Pd(PPh₃)₄ (3.18 g, 2.76 mmol) and K₂CO₃ (22.8 g, 165 mmol) were introduced to 1,4-dioxane/H₂O (190 mL/60 mL), and stirred for 2 hours at 100°C. The result was cooled to room temperature, filtered, and then washed with H₂O and MeOH to obtain Intermediate 395-1 (21.3 g, 86%) .

### Preparation of Compound 395

Intermediate 395-1 (7.00 g, 15.6 mmol), N-phenyl-[1,1'-biphenyl]-4-amine (4.02 g, 16.4 mmol), Pd₂dba₃ (0.71 g, 0.78 mmol), Xphos (0.74 g, 1.56 mmol) and t-BuONa (2.99 g, 31.2 mmol) were introduced to xylene, and stirred for 2 hours at 150°C. The result was cooled to room temperature and left unattended for 12 hours, and produced solids were filtered. The result was silica gel filtered and then vacuum concentrated, and produced solids were filtered to obtain Compound 395 (8.50 g, 83%).

Heterocyclic compounds corresponding to Chemical Formula 1 other than the compounds described in Preparation Examples 1 to 9 and Table 1 to Table 7 were also prepared in the same manner as in the methods described in the preparation examples described above.

Synthesis identification data for the compounds prepared above are as described in the following [Table 8] and [Table 9]. Table 8 shows measurement values of ¹H NMR (CDCl₃, 200 MHz), and Table 9 shows measurement values of FD-mass spectrometry (FD-MS: field desorption mass spectrometry).

**[Table 8]**

| **NO** | **¹H NMR (CDCl₃, 300 Mz)** |
|---|---|
| **6** | 8.37 (4H, ddd), 7.81-7.73 (4H, m), 7.60-7.37 (12H, m), 7.31-7.13 (6H, m), 7.11-6.97 (3H, m), 6.92 (1H, dd) |
| **11** | 8.35 (4H, ddd), 7.81-7.72 (6H, m), 7.61-7.37 (17H, m), 7.31-7.22 (5H, m), 7.15 (1H, dd), 6.88 (1H, dd), |
| **14** | 8.38 (4H, ddd), 8.10 (1H, dd), 7.79-7.71 (4H, m), 7.60-7.22 (20H, m), 7.20-7.05 (4H, m), 6.89 (1H, dd) |
| **18** | 8.41 (4H, ddd), 8.05 (1H, dd), 7.96 (1H, dd), 7.80-7.73 (4H, m), 7.61-7.20 (19H, m), 7.13 (1H, dd), 6.94-6.87 (2H, m) |
| **26** | 8.40 (2H, m), 7.92-7.83 (2H, m), 7.82-7.71 (4H, m), 7.57-7.13 (21H, m), 6.92 (1H, dd), 1.68 (6H, s) |
| **30** | 8.55 (1H, ddd), 8.39 (4H, dd), 8.20 (1H, dd), 7.81-7.72 (2H, m), 7.59-7.47 (8H, m), 7.38 (1H, dd), 7.28-7.07 (13H, m), 6.88 (1H, dd) |
| **38** | 8.38 (4H, ddd), 7.84-7.73 (2H, m), 7.61-7.44 (7H, m), 7.30-6.98 (21H, m), 6.92 (1H, dd) |
| **47** | 8.41 (4H, ddd), 8.22 (2H, dd), 7.87-7.72 (4H, m), 7.66-7.42 (22H, m), 7.28 (2H, dd) |
| **48** | 8.39 (4H, ddd), 7.81-773 (2H, m), 7.63-7.37 (17H, m), 7.31-7.21 (6H, m), 7.08 (2H, dd), 6.98 (1H, tt) |
| **52** | 8.40 (4H, ddd), 8.20 (1H, dd), 7.87 (1H, ddd), 7.80-7.72 (4H, m), 7.66-7.37 (22H, m), 7.30-7.22 (3H, m) |
| **53** | 8.38 (4H, ddd), 7.80-7.73 (6H, m), 7.64-7.36 (22H, m), 7.31-7.22 (6H, m) |
| **57** | 8.41 (4H, ddd), 8.01 (1H, dd), 7.80-7.73 (2H, m), 7.64-7.15 (21H, m), 7.08 (2H, ddd), 7.02-6.98 (2H, m) |
| **72** | 9.12 (1H, dd), 8.50 (1H, dd), 8.35 (2H, ddd), 8.13 (1H, ddd), 8.07 (1H, dd), 7.98 (1H, ddd), 7.81-7.73 (4H, m), 7.59-7.37 (11H, m), 7.31-6.98 (9H, m), 6.90 (1H, dd) |
| **73** | 9.11 (2H, dd), 8.48 (2H, dd), 8.16 (2H, ddd), 8.08 (2H, dd), 7.99 (2H, ddd), 7.82-7.71 (4H, m), 7.60-7.37 (10H, m), 7.31-6.99 (11H, m), 6.88 (1H, dd) |
| **75** | 8.40 (4H, ddd), 8.22 (2H, dd), 7.81-7.72 (6H, m), 7.61-7.37 (12H, m), 7.31-7.11 (8H, m), 7.08 (2H, ddd), 6.99 (1H, tt), 6.93 (1H, dd) |
| **76** | 8.40-8.35 (3H, m), 7.91 (1H, dd), 7.80-7.69 (7H, m), 7.63-7.39 (13H, m), 7.27-7.13 (6H, m), 7.10-6.99 (3H, m), 6.90 (1H, dd) |
| **81** | 8.36 (2H, ddd), 8.21 (1H, s), 7.96 (2H, ddd), 7.80-7.73 (4H, m), 7.57-7.40 (12H, m), 7.26-7.14 (6H, m), 7.10-6.99 (3H, m), 6.91 (1H, dd) |
| **87** | 8.24 (1H, s), 7.96 (4H, ddd), 7.80-7.73 (2H, m), 7.58-7.40 (12H, m), 7.28-7.13 (6H, m), 7.09-7.01 (3H, m), 6.92 (1H, dd) |
| **92** | 8.39 (4H, ddd), 8.25 (2H, dd), 7.79-7.75 (2H, m), 7.60-7.37 (12H, m), 7.30-7.16 (8H, m), 7.09-7.01 (3H, m), 6.99 (1H, dd) |
| **93** | 8.39 (4H, ddd), 7.93 (1H, dd), 7.80-7.69 (5H, m), 7.63-7.40 (13H, m), 7.29-7.14 (6H, m), 7.10-6.99 (3H, m), 6.90 (1H, dd) |
| **98** | 8.39 (2H, ddd), 8.10 (1H, dd), 7.96 (1H, dd), 7.80-7.75 (4H, m), 7.65-7.00 (23H, m), 6.90 (1H, dd) |
| **99** | 8.38 (2H, ddd), 8.28 (2H, dd), 8.11-7.98 (3H, m), 7.78-7.75 (2H m), 7.65-7.35 (13H, m), 7.28-7.15 (8H, m), 7.09-7.01 (3H, m), 6.90 (1H, dd) |
| **101** | 8.13 (1H, dd), 7.83-7.37 (18H, m), 7.29-7.04 (9H, m), 6.90 (1H, dd) |
| **109** | 7.88-7.74 (8H, m), 7.60-7.01 (20H, m), 6.92 (1H, dd) |
| **115** | 8.29 (2H, dd), 7.89-7.74 (10H, m), 7.59-7.22 (17H, m), 7.10-7.02 (3H, m), 6.90 (1H, dd) |
| **127** | 8.38 (4H, ddd), 8.20 (1H, d), 7.80-7.76 (4H, m), 7.59-7.40 (13H, m), 7.29-7.25 (4H, m), 7.10-6.95 (4H, m) |
| **132** | 8.39 (4H, m), 8.23 (1H, d), 7.81-7.73 (6H, m), 7.58-7.37 (18H, m), 7.29-7.26 (4H, m), 6.98 (1H, dd) |
| **156** | 8.41 (4H, ddd), 8.21 (1H, d), 7.80-7.74 (2H, m), 7.59-7.43 (8H, m), 7.26-7.23 (6H, m), 7.10-6.95 (14H, m) |
| **162** | 8.39 (4H, ddd), 7.74-7.41 (21H, m), 7.29-7.23 (6H, m), 7.10-6.99 (3H, m) |
| **183** | 8.37 (4H, ddd), 8.21 (1H, dd), 7.85-7.73 (4H, m), 7.70-7.44 (14H, m), 7.30-7.16 (5H, m), 7.12-7.02 (3H, m) |
| **186** | 9.12 (1H, dd), 8.51 (1H, dd), 8.37 (2H, ddd), 8.23-8.16 (2H, m), 8.08 (1H, dd), 8.00 (1H, ddd), 7.80-7.76 (2H, m), 7.62-7.41 (12H, m), 7.29-7.25 (4H, m), 7.11-6.99 (4H, m) |
| **229** | 8.39 (4H, ddd), 8.02 (1H, d), 7.79-7.73 (6H, m), 7.59-7.38 (18H, m), 7.28-7.25 (4H, m), 6.94 (1H, dd) |
| **259** | 8.37 (4H, ddd), 7.81-7.74 (6H, m), 7.56-7.40 (15H, m), 7.29-7.23 (6H, m), 7.09-6.98 (3H, m) |
| **301** | 8.39 (4H, ddd), 8.26 (2H, dd), 8.02 (1H, d), 7.81-7.75 (4H, m), 7.58-7.41 (13H, m), 7.28-7.24 (6H, m), 7.09-7.02 (3H, m), 6.90 (1H, dd) |
| **304** | 8.35 (2H, ddd), 8.03 (1H, d), 7.98 (1H, dd), 7.82-7.71 (6H, m), 7.55-7.22 (18H, m), 7.10-7.00 (3H, m), |
| **308** | 8.35 (2H, ddd), 8.24 (2H, dd), 8.11-7.98 (4H, m), 7.80-7.73 (4H, m), 7.66-7.40 (14H, m), 7.28-7.22 (6H, m), 7.10-7.01 (3H, m), 6.92 (1H, dd) |
| **325** | 8.26 (4H, ddd), 7.78-7.74 (6H, m), 7.62-7.43 (18H, m), 7.27 (4H, dd), 7.20 (1H, dd), 6.99 (1H, dd) |
| **332** | 8.46 (1H, dd), 8.37 (4H, ddd), 7.97 (1H, dd), 7.89-7.75 (6H, m), 7.56-7.42 (15H, m), 7.32-7.25 (3H, m), 7.20 (1H, dd), 6.99 (1H, dd) |
| **337** | 8.38 (4H, ddd), 7.90-7.86 (2H, m), 7.77-7.75 (4H, m), 7.64-7.28 (19H, m), 7.16 (1H, dd), 6.98 (1H, dd), 1.69 (6H, s) |
| **347** | 8.37 (4H, ddd), 8.08 (1H, dd), 8.02 (1H, dd), 7.79-7.74 (4H, m), 7.57 (15H, m), 7.28-7.22 (6H, m), 7.09-7.01 (3H, m) |
| **355** | 8.56 (1H, dd), 8.28 (4H, ddd), 8.18 (1H, dd), 8.07 (1H, dd), 8.02 (1H, dd), 7.78-7.76 (2H, m), 7.70 (2H, dd), 7.57-7.39 (12H, m), 7.28-6.99 (13H, m) |
| **395** | 8.40 (4H, ddd), 8.21 (1H, d), 7.98 (1H, d), 7.76 (4H, ddd), 7.65 (1H, dd), 7.56-7.39 (18H, m), 7.28-7.25 (5H, m) |

**[Table 9]**

| **Compound** | FD-MS | **Compound** | FD-MS |
|---|---|---|---|
| **6** | m/z=642.24 (C45H30N4O=642.76) | **99** | m/z=768.29 (C55H36N4O=768.92) |
| **11** | m/z=718.27 (C51H34N4O=718.86) | **101** | m/z=615.23 (C44H29N3O=615.74) |
| **14** | m/z=718.27 (C51H34N4O=718.86) | **109** | m/z=671.20 (C46H29N3OS=671.82) |
| **18** | m/z=732.25 (C51H32N4O2=732.84) | **115** | m/z=747.23 (C52H33N3OS=747.92) |
| **26** | m/z=758.30 (C54H38N4O=758.93) | **127** | m/z=642.24 (C45H30N4O=642.76) |
| **30** | m/z=731.27 (C51H33N5O=731.86) | **132** | m/z=718.27 (C51H34N4O=718.86) |
| **38** | m/z=733.28 (C51H35N5O=733.88) | **156** | m/z=733.28 (C51H35N5O=733.88) |
| **47** | m/z=742.27 (C53H34N4O=742.88) | **162** | m/z=718.27 (C51H34N4O=718.86) |
| **48** | m/z=718.27 (C51H34N4O=718.86) | **183** | m/z=692.26 (C49H32N4O=692.82) |
| **52** | m/z=768.29 (C55H36N4O=768.92) | **186** | m/z=692.26 (C49H32N4O=692.82) |
| **53** | m/z=794.30 (C57H38N4O=794.96) | **229** | m/z=718.27 (C51H34N4O=718.86) |
| **57** | m/z=732.25 (C51H32N4O2=732.84) | **259** | m/z=718.27 (C51H34N4O=718.86) |
| **72** | m/z=692.26 (C49H32N4O=692.82) | **301** | m/z=718.27 (C51H34N4O=718.86) |
| **73** | m/z=742.27 (C53H34N4O=742.88) | **304** | m/z=732.25 (C51H32N4O2=732.84) |
| **75** | m/z=718.27 (C51H34N4O=718.86) | **308** | m/z=768.29 (C55H36N4O=768.92) |
| **76** | m/z=718.27 (C51H34N4O=718.86) | **325** | m/z=718.27 (C51H34N4O=718.86) |
| **81** | m/z=641.25 (C46H31N3O=641.77) | **332** | m/z=748.23 (C51H32N4OS=748.90) |
| **87** | m/z=641.25 (C46H31N3O=641.77) | **337** | m/z=758.30 (C54H38N4O=758.93) |
| **92** | m/z=718.27 (C51H34N4O=718.86) | **347** | m/z=718.27 (C51H34N4O=718.86) |
| **93** | m/z=718.27 (C51H34N4O=718.86) | **355** | m/z=807.30 (C57H37N5O=807.96) |
| **98** | m/z=732.25 (C51H32N4O2=732.84) | **395** | m/z=734.25 (C51H34N4S=734.92) |

### <Experimental Example 1>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO (ultraviolet ozone) treatment was conducted for 5 minutes using UV in a UV (ultraviolet) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. Specifically, the light emitting layer was deposited to a thickness of 500 Å using each compound described in Examples 1 to 42 and Comparative Examples 1 to 6 of the following Table 10 as a red host of the light emitting layer, and doping (piq)₂(Ir)(acac), a red phosphorescent dopant, by 3 wt% to the red host. After that, bathocuproine (hereinafter, BCP) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 1 to 42 and Comparative Examples 1 to 6 manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. T90 means a lifetime (unit: h, time), time taken to become 90% with respect to initial luminance.

Measured properties of the organic light emitting devices are as shown in the following Table 10.

**[Table 10]**

| | Comp ound | Threshold Voltage (Von) | Driving Voltage (Vop) | Efficie ncy (cd/A) | Color Coordinate (x, Y) | Life time (T₉₀) |
|---|---|---|---|---|---|---|
| Comparative Example 1 | A | 2.60 | 6.04 | 10.2 | (0.676, 0.324) | 7 |
| Comparative Example 2 | B | 2.72 | 6.31 | 8.9 | (0.675, 0.325) | 5 |
| Comparative Example 3 | C | 2.91 | 6.55 | 6.7 | (0.674, 0.326) | 3 |
| Comparative Example 4 | D | 2.83 | 6.48 | 8.2 | (0.678, 0.322) | 9 |
| Comparative Example 5 | E | 2.75 | 6.41 | 9.5 | (0.680, 0.319) | 8 |
| Comparative Example 6 | F | 2.82 | 6.45 | 8.8 | (0.681, 0.319) | 7 |
| Example 1 | 6 | 2.30 | 5.12 | 50.3 | (0.679, 0.320) | 65 |
| Example 2 | 11 | 2.42 | 5.03 | 50.0 | (0.678, 0.322) | 68 |
| Example 3 | 14 | 2.61 | 5.54 | 45.6 | (0.681, 0.319) | 60 |
| Example 4 | 18 | 2.37 | 5.31 | 61.5 | (0.680, 0.320) | 83 |
| Example 5 | 26 | 2.32 | 5.25 | 48.7 | (0.682, 0.318) | 61 |
| Example 6 | 30 | 2.30 | 5.11 | 65.5 | (0.677, 0.323) | 85 |
| Example 7 | 38 | 2.31 | 5.08 | 48.9 | (0.678, 0.321) | 65 |
| Example 8 | 47 | 2.55 | 5.49 | 45.9 | (0.679, 0.319) | 62 |
| Example 9 | 48 | 2.34 | 5.07 | 49.5 | (0.679, 0.319) | 71 |
| Example 10 | 52 | 2.42 | 5.22 | 48.9 | (0.680, 0.319) | 68 |
| Example 11 | 53 | 2.32 | 5.05 | 48.8 | (0.681, 0.320) | 69 |
| Example 12 | 57 | 2.52 | 5.45 | 63.2 | (0.682, 0.318) | 82 |
| Example 13 | 72 | 2.35 | 5.10 | 47.9 | (0.683, 0.317) | 63 |
| Example 14 | 73 | 2.38 | 5.17 | 48.2 | (0.682, 0.319) | 65 |
| Example 15 | 75 | 2.28 | 5.00 | 49.5 | (0.679, 0.320) | 66 |
| Example 16 | 76 | 2.35 | 5.11 | 48.7 | (0.680, 0.320) | 63 |
| Example 17 | 81 | 2.57 | 5.52 | 43.2 | (0.677, 0.322) | 50 |
| Example 18 | 87 | 2.49 | 5.47 | 45.5 | (0.679, 0.320) | 54 |
| Example 19 | 92 | 2.34 | 5.18 | 47.4 | (0.681, 0.319) | 67 |
| Example 20 | 93 | 2.37 | 5.20 | 47.1 | (0.678, 0.321) | 65 |
| Example 21 | 98 | 2.55 | 5.43 | 47.5 | (0.680, 0.320) | 60 |
| Example 22 | 99 | 2.35 | 5.18 | 48.5 | (0.681, 0.319) | 62 |
| Example 23 | 101 | 2.48 | 5.41 | 46.1 | (0.676, 0.323) | 58 |
| Example 24 | 109 | 2.45 | 5.37 | 47.5 | (0.678, 0.321) | 57 |
| Example 25 | 115 | 2.43 | 5.35 | 48.1 | (0.680, 0.320) | 55 |
| Example 26 | 127 | 2.28 | 5.05 | 40.1 | (0.682, 0.318) | 42 |
| Example 27 | 132 | 2.26 | 5.09 | 40.3 | (0.681, 0.319) | 44 |
| Example 28 | 156 | 2.24 | 5.05 | 35.6 | (0.680, 0.320) | 39 |
| Example 29 | 162 | 2.31 | 5.09 | 35.0 | (0.681, 0.319) | 44 |
| Example 30 | 183 | 2.42 | 5.40 | 36.0 | (0.679, 0.320) | 41 |
| Example 31 | 186 | 2.29 | 5.00 | 35.9 | (0.680, 0.319) | 43 |
| Example 32 | 229 | 2.28 | 4.98 | 35.7 | (0.683, 0.317) | 46 |
| Example 33 | 259 | 2.29 | 4.98 | 35.2 | (0.681, 0.319) | 50 |
| Example 34 | 301 | 2.31 | 5.05 | 35.8 | (0.677, 0.323) | 49 |
| Example 35 | 304 | 2.35 | 5.15 | 36.7 | (0.681, 0.319) | 43 |
| Example 36 | 308 | 2.30 | 4.99 | 36.5 | (0.680, 0.320) | 45 |
| Example 37 | 325 | 2.42 | 5.41 | 33.2 | (0.679, 0.320) | 32 |
| Example 38 | 332 | 2.49 | 5.57 | 42.4 | (0.677, 0.323) | 49 |
| Example 39 | 337 | 2.48 | 5.52 | 31.1 | (0.681, 0.319) | 36 |
| Example 40 | 347 | 2.48 | 5.48 | 32.1 | (0.677, 0.322) | 30 |
| Example 41 | 355 | 2.45 | 5.43 | 51.7 | (0.679, 0.321) | 58 |
| Example 42 | 395 | 2.51 | 5.61 | 28.7 | (0.680, 0.320) | 29 |

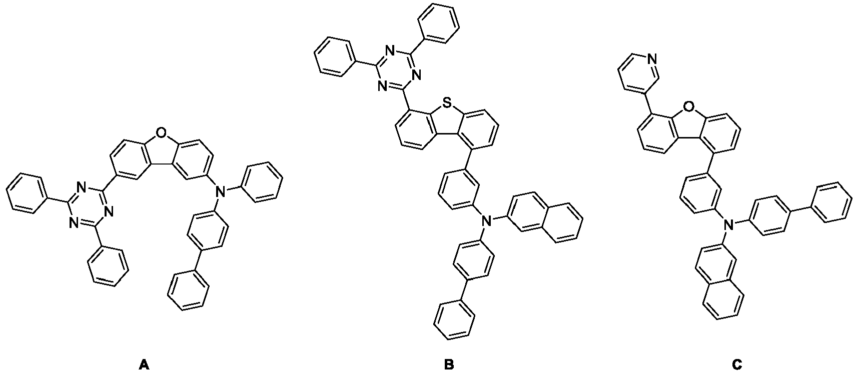

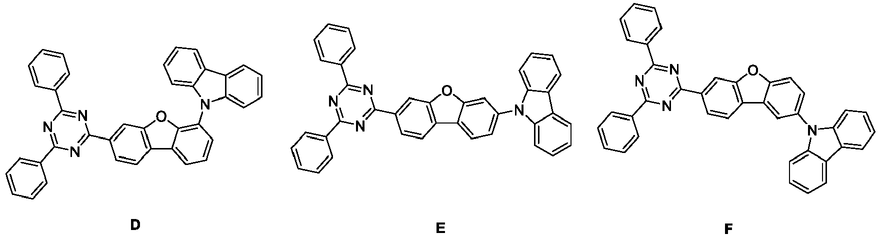

The heterocyclic compound according to the present application has proper molecular weight and band gap to be used in a light emitting layer of an organic light emitting device while having high thermal stability. A proper molecular weight facilitates formation of a light emitting layer of an organic light emitting device, and a proper band gap prevents loss of electrons and holes in the light emitting layer helping with effective formation of a recombination zone.

In other words, it was identified that, in the compound having an arylamine group substituted at a proper position as the heterocyclic compound according to the present application, strong hole transfer (HT) properties of the arylamine group substituted at the position resolved a hole blocking (hole trap) phenomenon occurring in the dopant. This was identified through the HOMO/LUMO data of the following Table 11 and Table 12.

**[Table 11]**

| | Compound | HOMO | | Eg | LUMO | |
|---|---|---|---|---|---|---|
| Exam ple 1-1 | 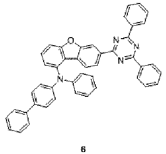 | - 5.4 6 | 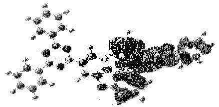 | 3. 15 | - 2.3 1 | 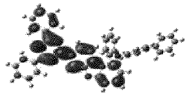 |
| Exam ple 1-2 | 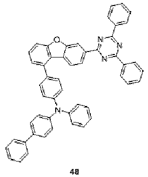 | - 5.3 4 | 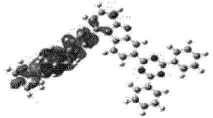 | 3. 03 | - 2.3 1 | 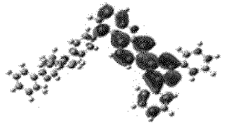 |
| Exam ple 1-3 | 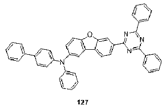 | - 5.3 0 | 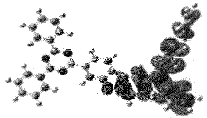 | 2. 95 | - 2.3 5 | 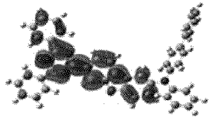 |
| Exam ple 1-4 | 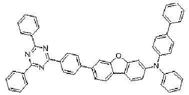 | - 5.2 8 | 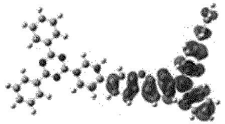 | 3. 00 | - 2.2 8 | 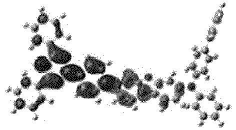 |
| Exam ple 1-5 | 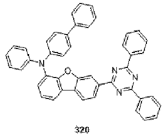 | - 5.3 6 | 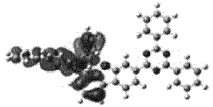 | 3. 03 | - 2.3 3 | 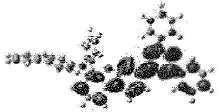 |
| Comp arat ive Exam ple 1-1 | 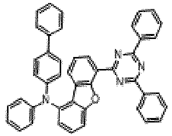 | - 5.4 3 | 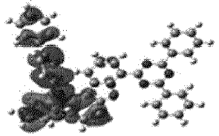 | 3. 20 | - 2.2 3 | 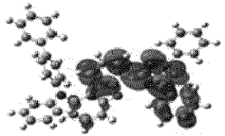 |
| Comp arat ive Exam ple 1-2 | 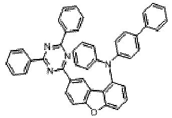 | - 5.3 9 | 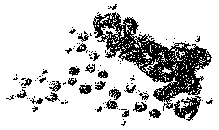 | 3. 26 | - 2.1 4 | 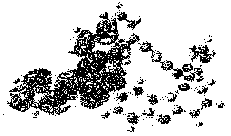 |

**[Table 12]**

| | S₁ | T₁ | Dipole Moment |
|---|---|---|---|
| Example 1-1 | 2.69 | 2.46 | 1.08 |
| Example 1-2 | 2.67 | 2.51 | 1.60 |
| Example 1-3 | 2.55 | 2.38 | 0.55 |
| Example 1-4 | 2.70 | 2.38 | 1.52 |
| Example 1-5 | 2.62 | 2.42 | 0.60 |
| Comparative Example 1-1 | 2.76 | 2.61 | 1.20 |
| Comparative Example 1-2 | 2.82 | 2.74 | 0.93 |

A compound having an electron transfer (ET) group substituted at a proper position as the heterocyclic compound according to the present application has a lower T1 value compared to a compound having an electron transfer group substituted at another position. A general red dopant has a low T1 value, and it is considered that efficiency increases by energy transfer from a host to a dopant readily occurring. In addition, a threshold voltage may be adjusted depending on the tendency of the electron transfer (ET) group. Specifically, when the core structure of the heterocyclic compound according to the present application is substituted with amine, the heteroatom of the amine group enhances a hole injection ability of the hole transfer group increasing efficiency and lifetime.

It was identified that, when the core structure of the heterocyclic compound according to the present application is substituted with amine, the heteroatom of the amine group enhanced a hole injection ability of the hole transfer group increasing efficiency and lifetime.

### <Experimental Example 2>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO (ultraviolet ozone) treatment was conducted for 5 minutes using UV in a UV (ultraviolet) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. Specifically, the light emitting layer was deposited to a thickness of 500 Å using each compound described in Examples 1 to 9 of the following Table 13 as a red host of the light emitting layer, and doping (piq)₂(Ir)(acac), a red phosphorescent dopant, by 3 wt% to the red host. After that, bathocuproine (hereinafter, BCP) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 1 to 9 of Table 13 manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. T90 means a lifetime (unit: h, time), time taken to become 90% with respect to initial luminance.

Measured properties of the organic light emitting devices are as shown in the following Table 13.

**[Table 13]**

| | Compound | Ratio (P/N ) | Threshold Voltage (Von) | Driving Voltage (Vₒₚ) | Efficiency (cd/A ) | Color Coordinate (x, y) | Life time (T₉₀ ) |
|---|---|---|---|---|---|---|---|
| Comparative Example 1 | A:B | 1:2 | 2.60 | 6.13 | 9.5 | (0.678, 0.322) | 9 |
| Comparative Example 2 | | 1:1 | 2.55 | 6.09 | 9.9 | (0.680, 0.320) | 8 |
| Comparative Example 3 | | 2:1 | 2.53 | 6.04 | 10.0 | (0.677, 0.323) | 6 |
| Example 1 | Comp ound 6:B | 1:2 | 2.25 | 4.90 | 85.1 | (0.679, 0.320) | 101 |
| Example 2 | | 1:1 | 2.28 | 4.93 | 87.1 | (0.680, 0.320) | 98 |
| Example 3 | | 2:1 | 2.33 | 5.01 | 89.2 | (0.678, 0.322) | 95 |
| Example 4 | Comp ound 87:C ompo und 6 | 1:2 | 2.51 | 5.57 | 88.6 | (0.681, 0.319) | 98 |
| Example 5 | | 1:1 | 2.48 | 5.52 | 91.5 | (0.678, 0.322) | 102 |
| Example 6 | | 2:1 | 2.43 | 5.48 | 93.7 | (0.680, 0.320) | 108 |
| Example 7 | Comp ound 81:C ompo und 6 | 1:2 | 2.59 | 5.61 | 86.9 | (0.681, 0.319) | 100 |
| Example 8 | | 1:1 | 2.53 | 5.58 | 87.1 | (0.677, 0.323) | 105 |
| Example 9 | | 2:1 | 2.50 | 5.51 | 89.2 | (0.682, 0.318) | 112 |

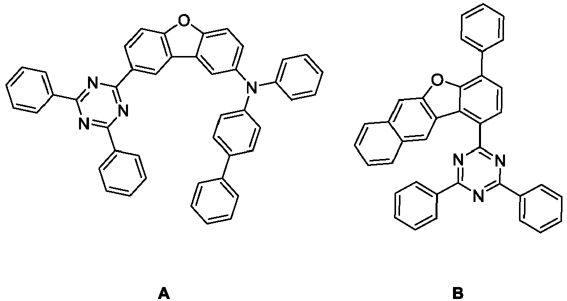

As seen from the results of Table 13, it was identified that, when mixing and depositing the heterocyclic compound according to the present application and the heterocyclic compounds of Compounds A and B as the organic material layer of the organic light emitting device, threshold voltage and driving voltage were able to be properly adjusted, and an effect of efficiency or lifetime was improved in the organic light emitting device.

Specifically, it is identified that the device lifetime is improved when combining the heterocyclic compound according to the present application with a specific N-type host compound like Compound A. Combining an N-type host compound having strong electron transfer properties with a P-type host compound having strong hole transfer properties performs a role of achieving a charge balance in the device, which significantly improves the lifetime.

In addition, when combining Compound 81 or 87 that weakens an ability of the electron transfer group with Compound 6 that has a strong ability of the electron transfer group, the compound that weakens an ability of the electron transfer group performs a role of an electron blocking layer (EBL) improving device efficiency and lifetime, and a proper threshold voltage may be formed by the increased LUMO level.

## Claims

1. A heterocyclic compound represented by the following Chemical Formula 1: 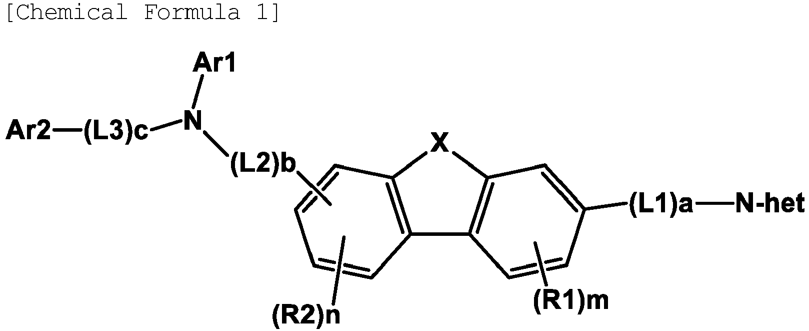 wherein, in Chemical Formula 1,
R1 and R2 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and - SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
N-het is a monocyclic or polycyclic C2 to C60 heterocyclic group substituted or unsubstituted and comprising one or more Ns;
X is O; or S;
L1 to L3 are the same as or different from each other, and each independently a direct bond; a substituted or unsubstituted C6 to C60 arylene group; or a substituted or unsubstituted C2 to C60 heteroarylene group;
Ar1 and Ar2 are the same as or different from each other, and each independently a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - NR201R202; -P(=O)RR'; or -SiRR'R";
a to c are an integer of 0 to 4;
m and n are an integer of 0 to 3;
when a to c, m and n are 2 or greater, substituents in the parentheses are the same as or different from each other; and
R201, R202, R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

2. The heterocyclic compound of Claim 1, wherein Chemical Formula 1 is represented by any one of the following Chemical Formulae 2 to 5: 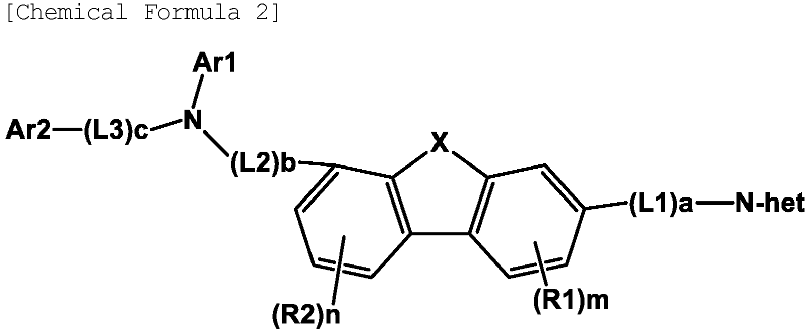 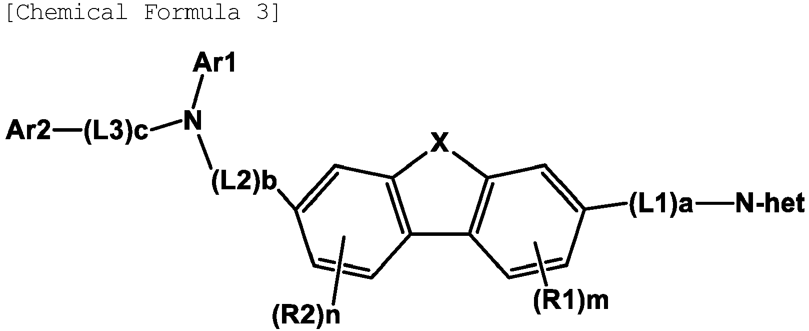 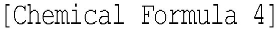 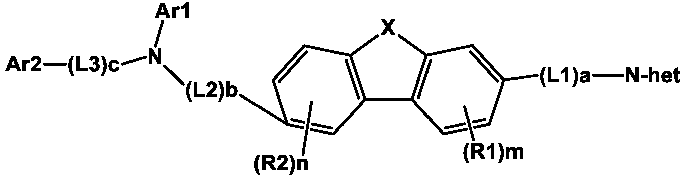 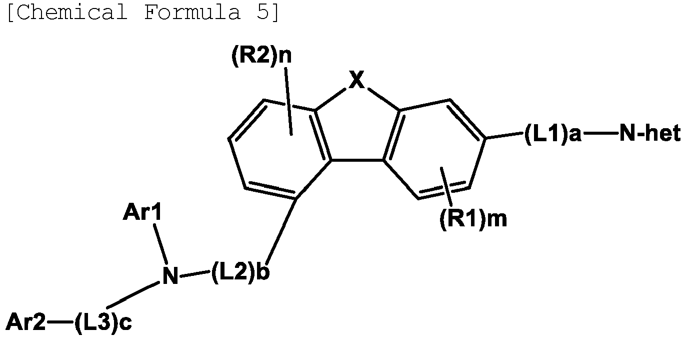 in Chemical Formulae 2 to 5,
each substituent has the same definition as in Chemical Formula 1.

3. The heterocyclic compound of Claim 1, wherein N-Het is represented by any one of the following Chemical Formulae 1-1 to 1-5: 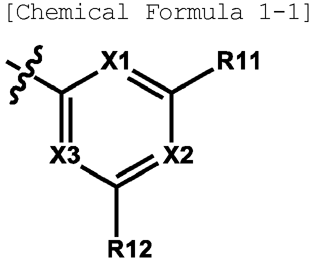 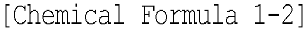 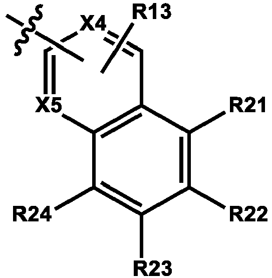 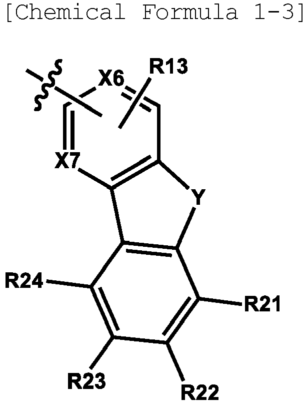 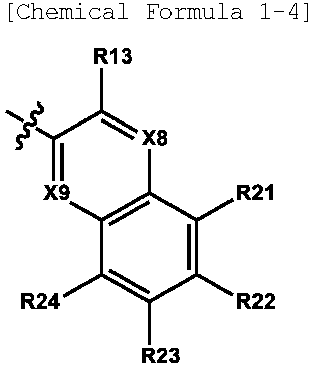 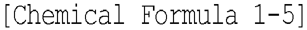 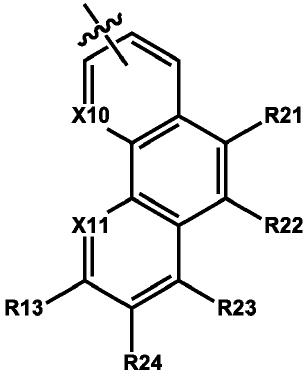 in Chemical Formulae 1-1 to 1-5,
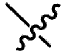 means a position linked to Chemical Formula 1;
X1 to X3 are the same as or different from each other, and each independently N or CRa;
X4 and X5 are the same as or different from each other, and each independently N or CRa;
X6 and X7 are the same as or different from each other, and each independently N or CRa;
X8 and X9 are the same as or different from each other, and each independently N or CRa;
X10 and X11 are the same as or different from each other, and each independently N or CRa;
at least one of X1 to X3, at least one of X6 and X7, at least one of X8 and X9 and at least one of X10 and X11 are N;
Y is O; or S;
R11 to R13 are the same as or different from each other, and each independently hydrogen; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group;
R21 to R24 are the same as or different from each other, and each independently hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group; and
Ra is hydrogen; a substituted or unsubstituted C6 to C60 aryl group; or a substituted or unsubstituted C2 to C60 heteroaryl group.

4. The heterocyclic compound of Claim 1, wherein 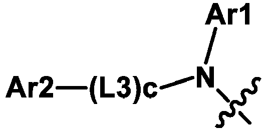 of Chemical Formula 1 is represented by any one of the following Chemical Formulae 1-1-1 to 1-1-5: 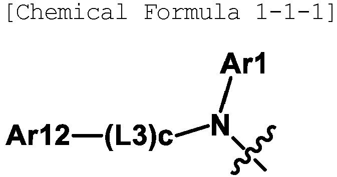 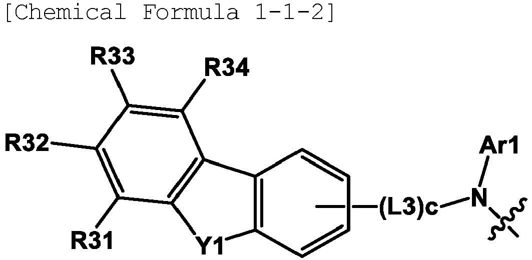 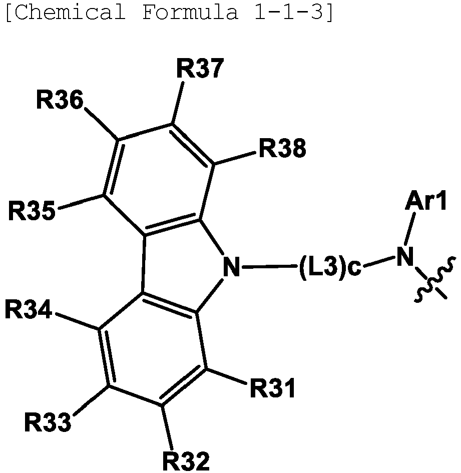 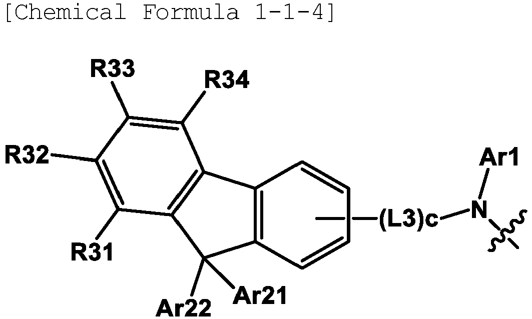 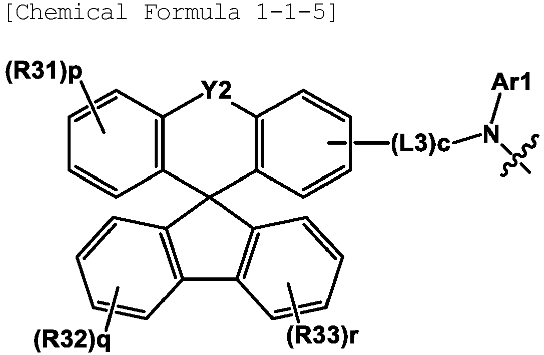 in Chemical Formulae 1-1-1 to 1-1-5,
Ar1, L3 and c have the same definitions as in Chemical Formula 1, and 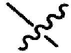 means a position linked to Chemical Formula 1;
Ar12 is a substituted or unsubstituted C6 to C60 aryl group; or -NR201R202;
Y1 is O; S; or NRb;
Y2 is O; or S;
Ar21 and Ar22 are the same as or different from each other, and each independently a substituted or unsubstituted C1 to C60 alkyl group; or a substituted or unsubstituted C6 to C60 aryl group, or two groups adjacent to each other bond to each other to form a substituted or unsubstituted C6 to C60 aromatic hydrocarbon ring;
R31 to R38 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group;
R201 and R202 have the same definitions as in Chemical Formula 1;
Rb is a substituted or unsubstituted C1 to C60 alkyl group; a substituted or unsubstituted C6 to C60 aryl group; or a substituted or unsubstituted C2 to C60 heteroaryl group; and
p, q and r are an integer of 0 to 4.

5. The heterocyclic compound of Claim 1, wherein R1 and R2 are hydrogen.

6. The heterocyclic compound of Claim 1, wherein Chemical Formula 1 is represented by any one of the following compounds:

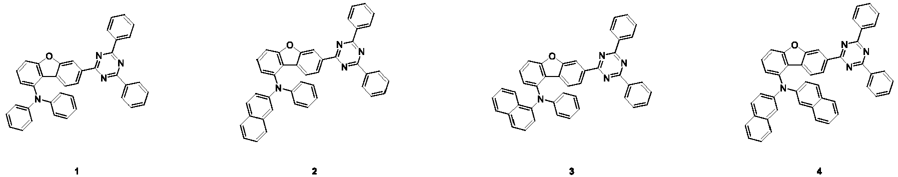

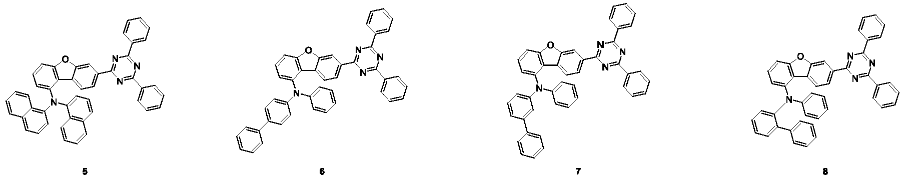

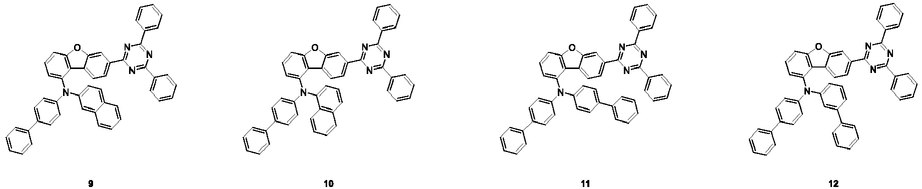

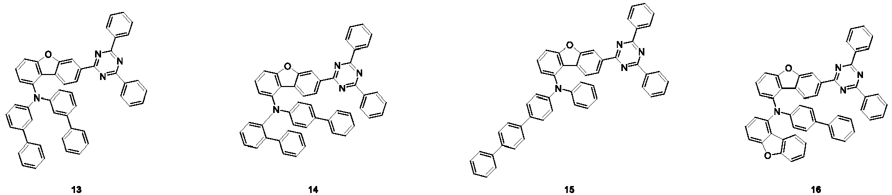

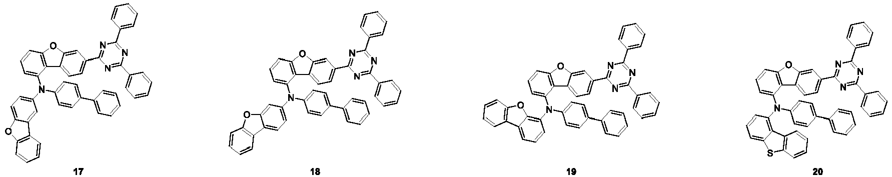

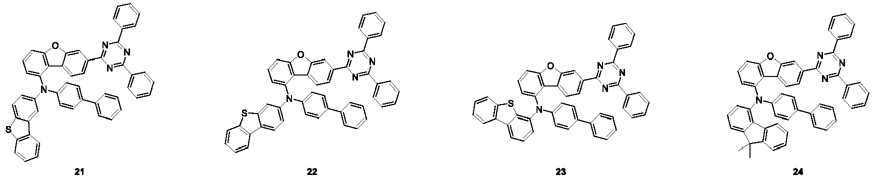

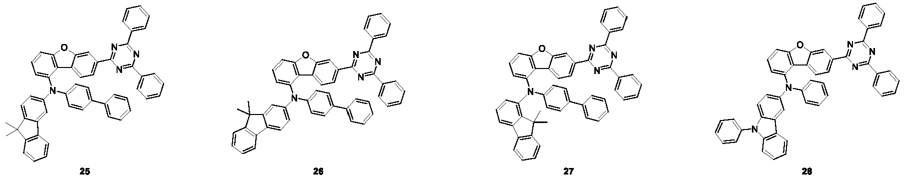

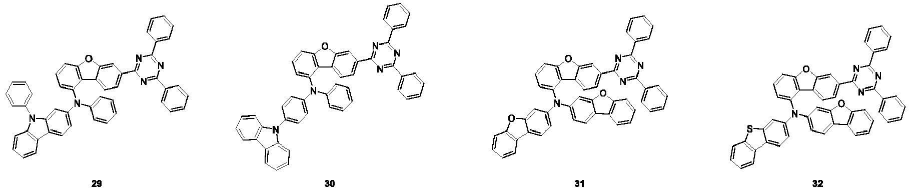

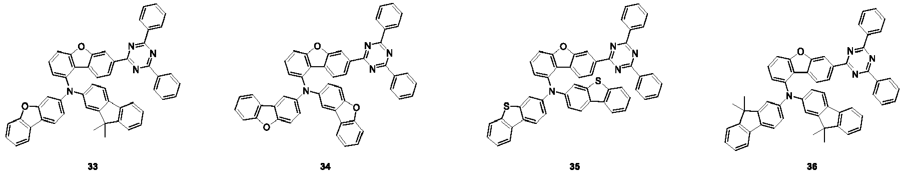

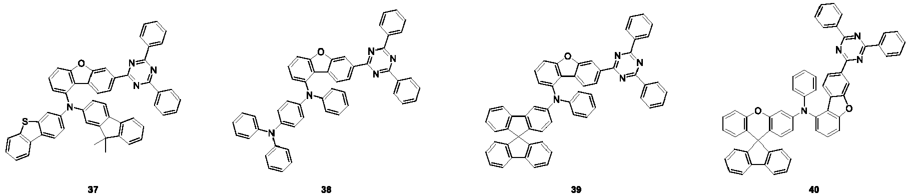

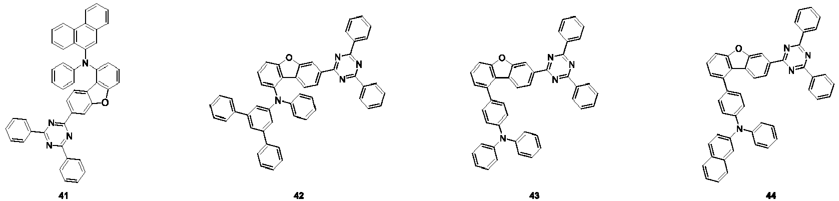

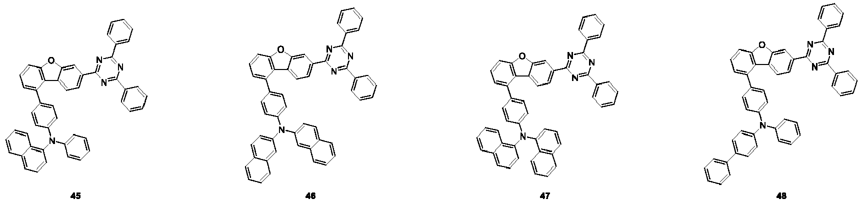

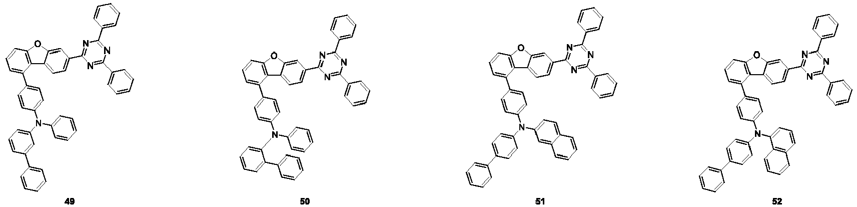

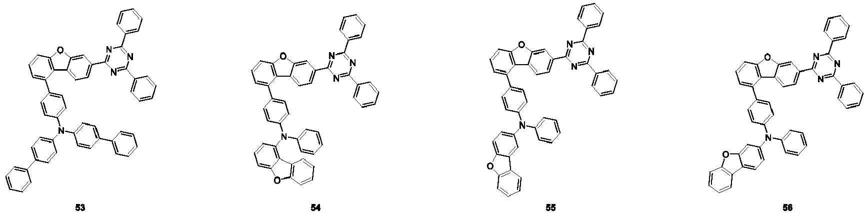

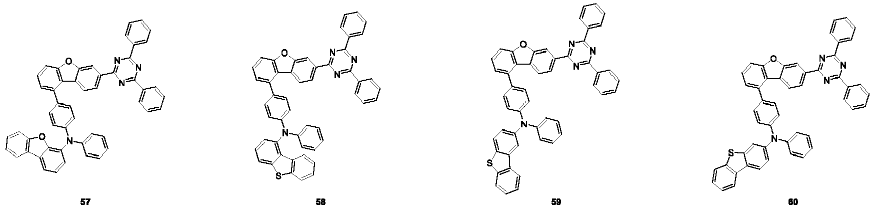

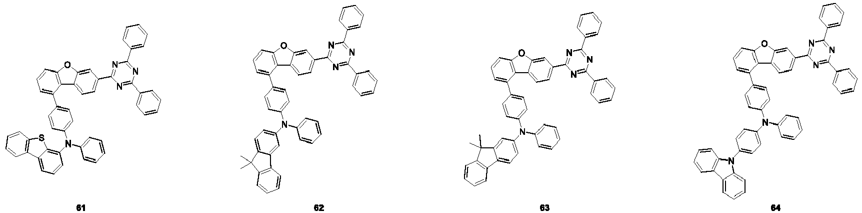

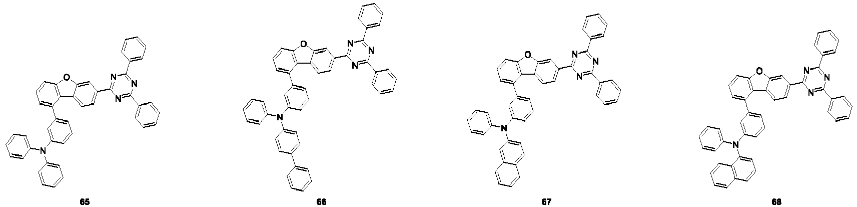

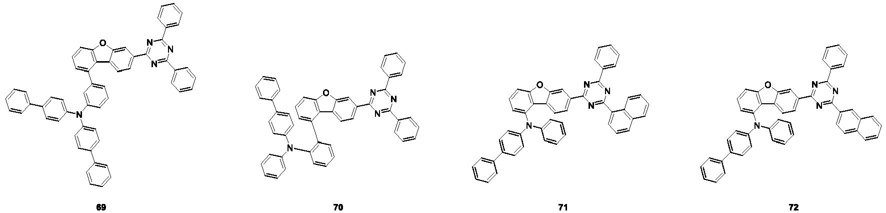

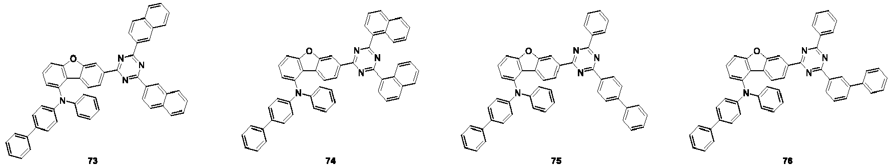

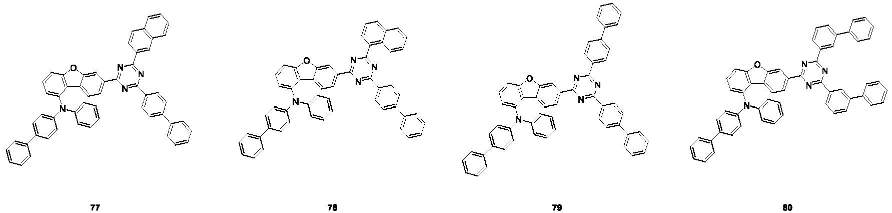

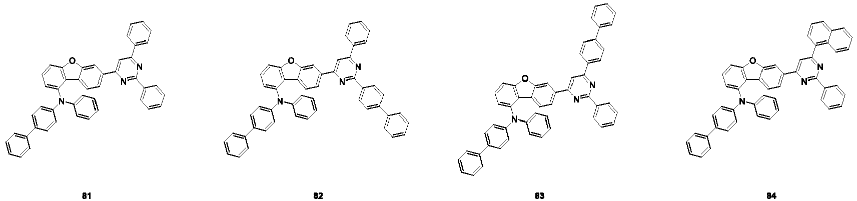

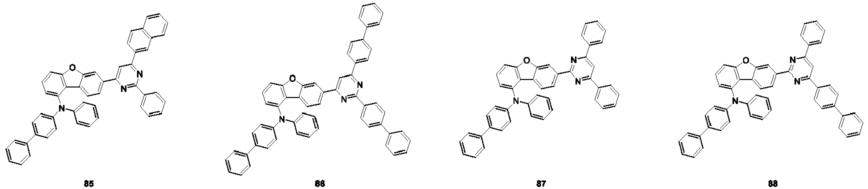

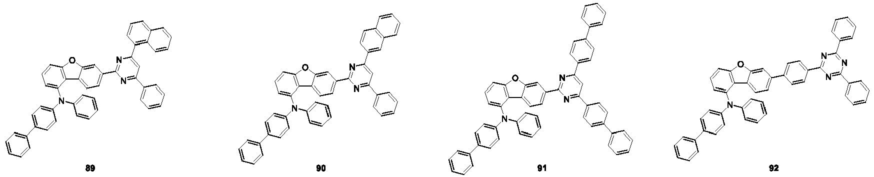

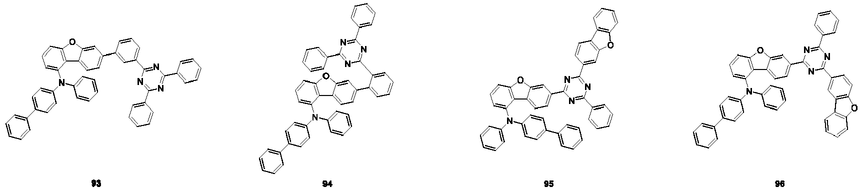

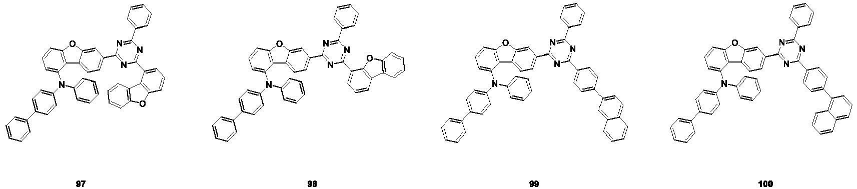

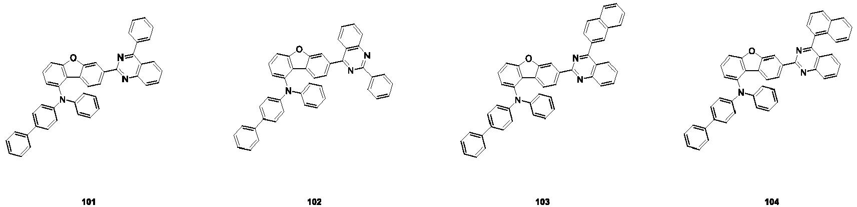

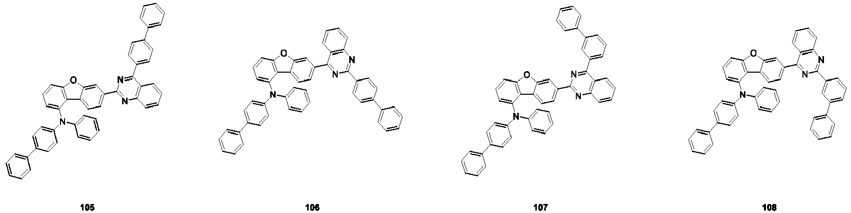

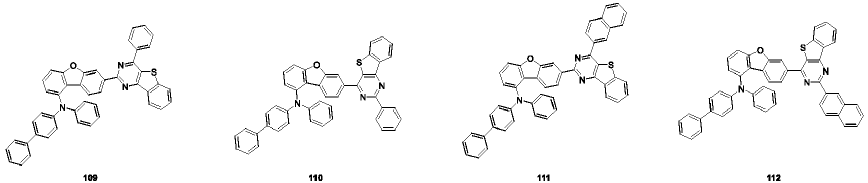

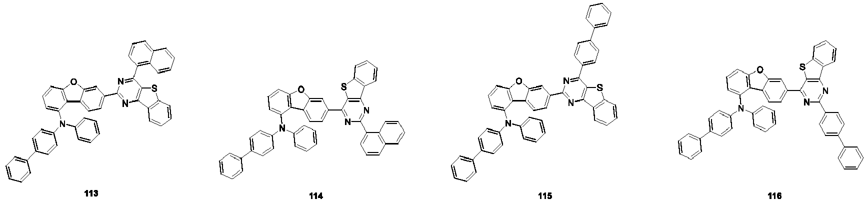

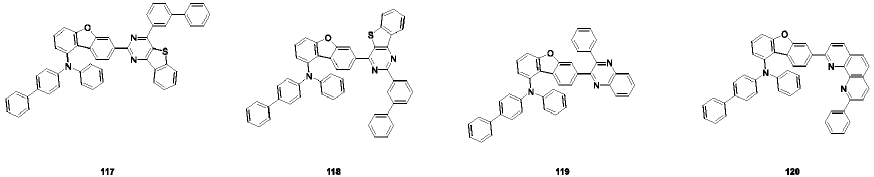

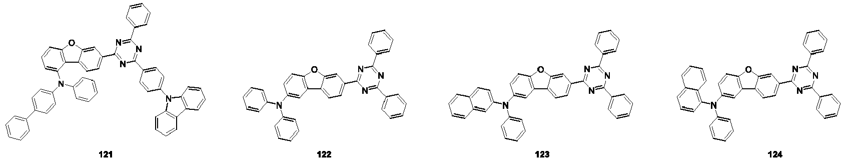

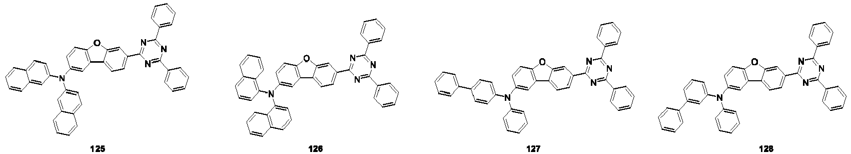

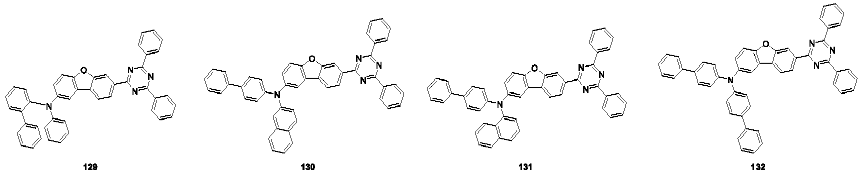

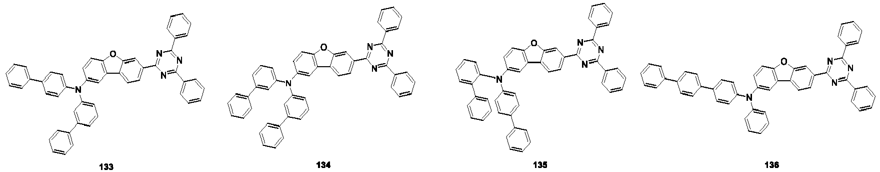

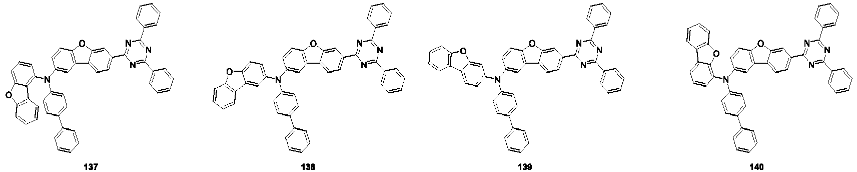

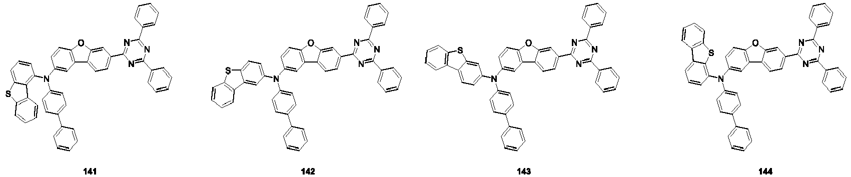

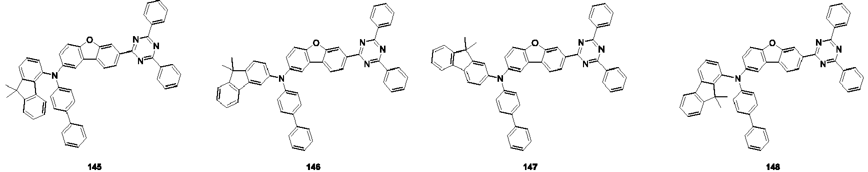

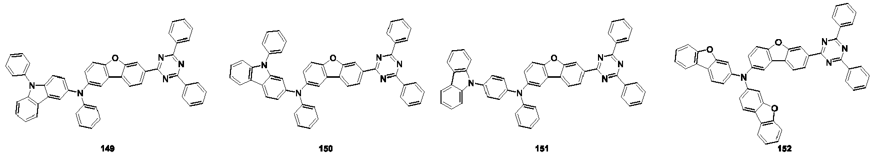

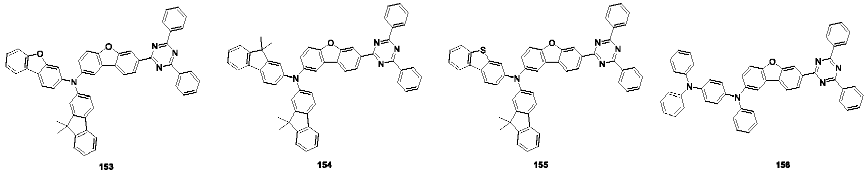

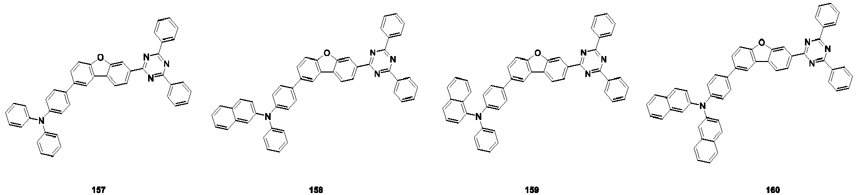

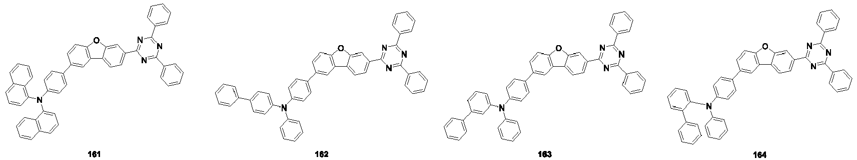

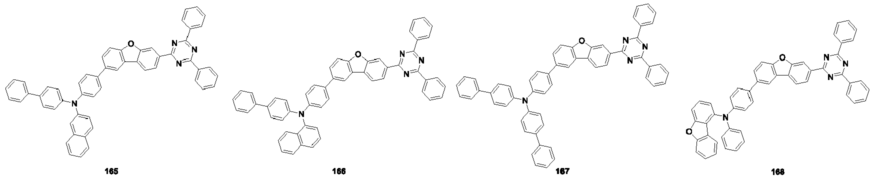

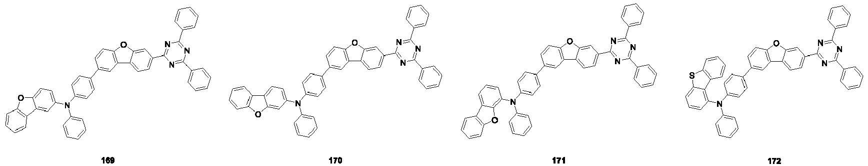

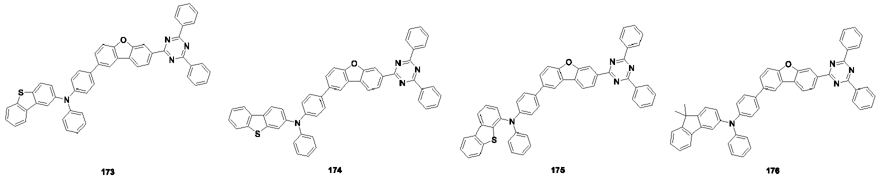

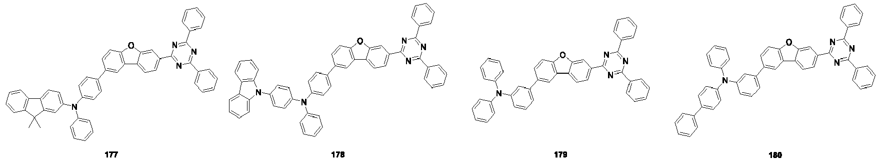

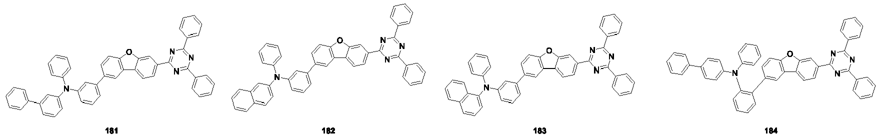

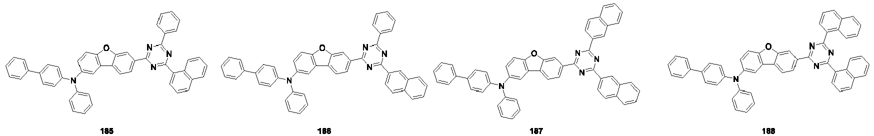

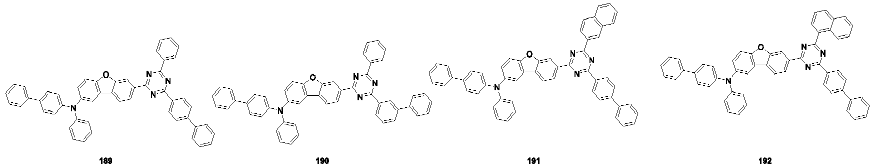

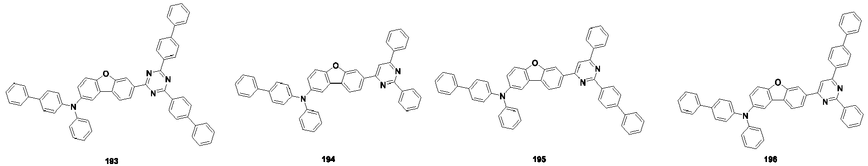

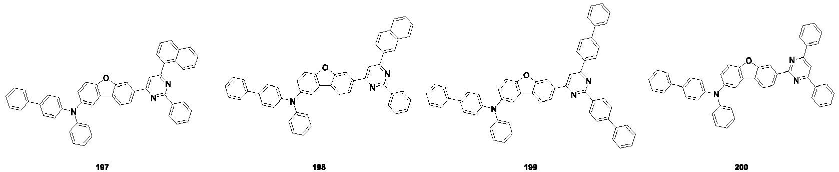

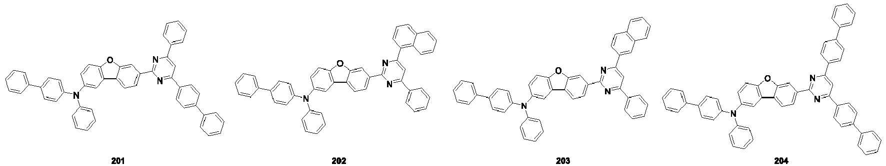

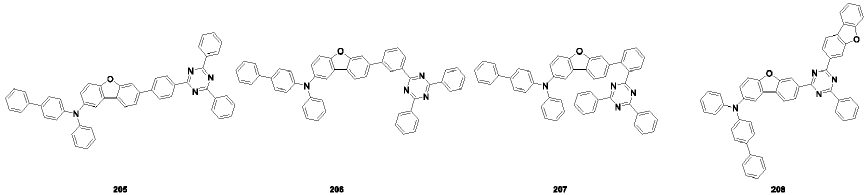

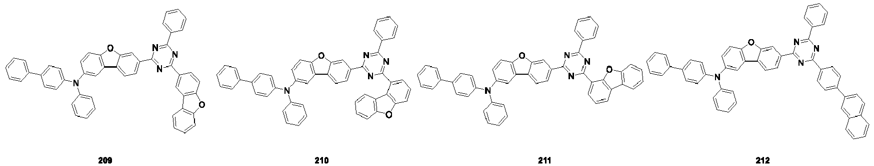

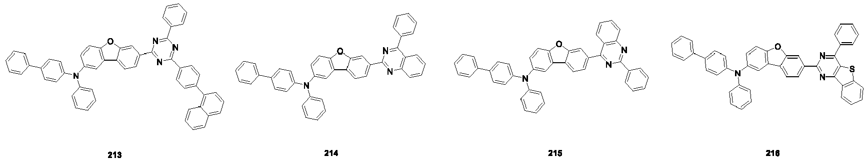

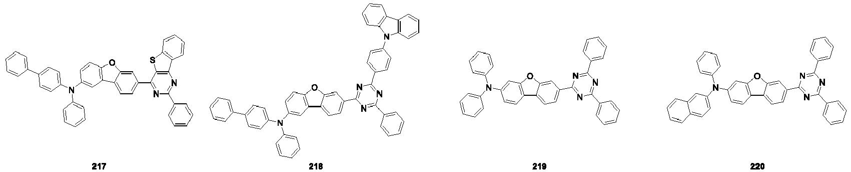

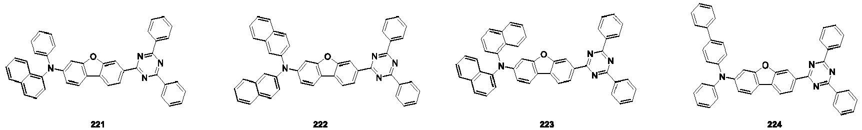

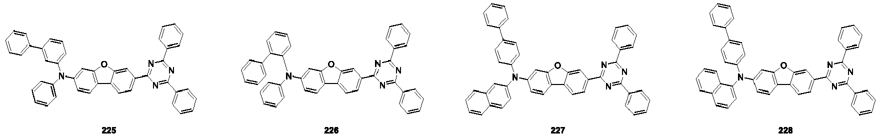

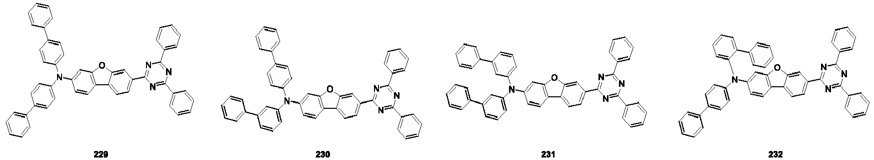

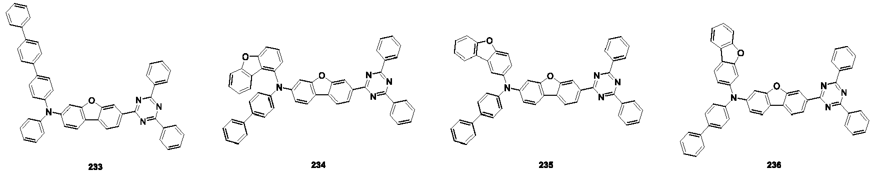

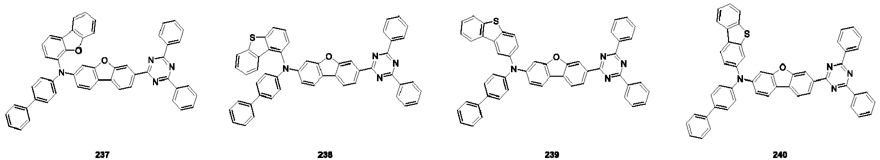

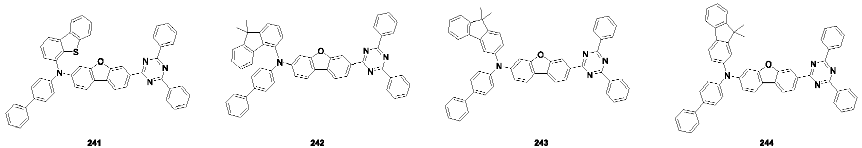

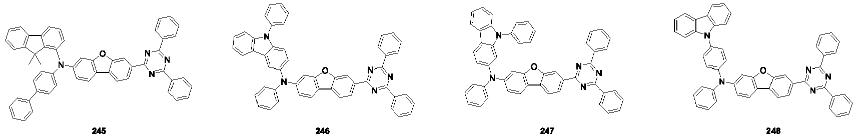

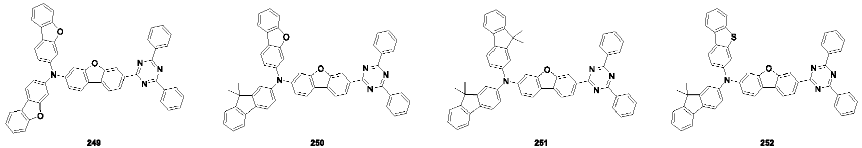

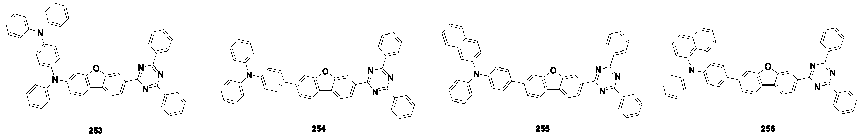

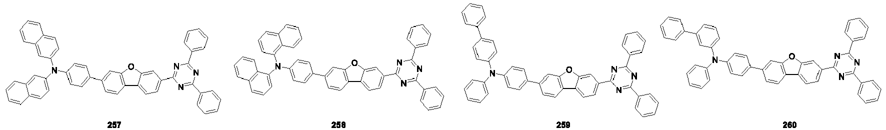

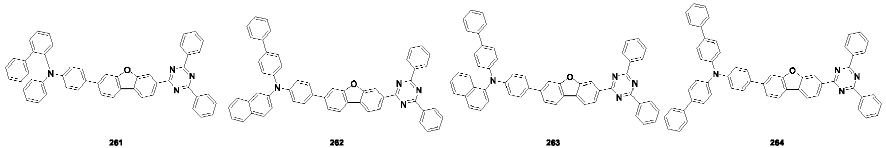

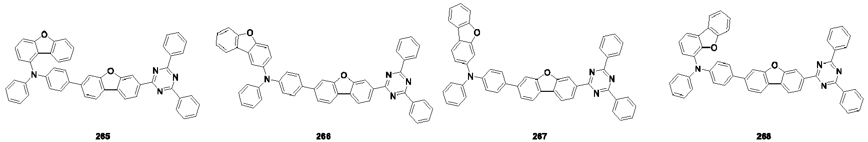

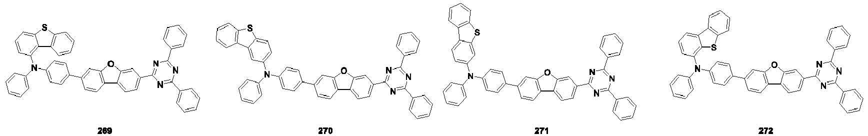

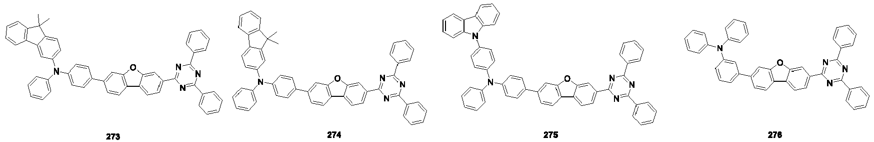

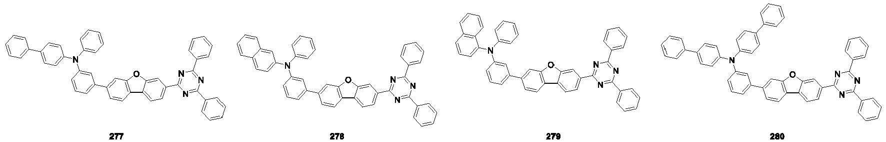

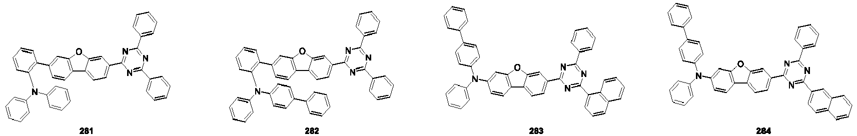

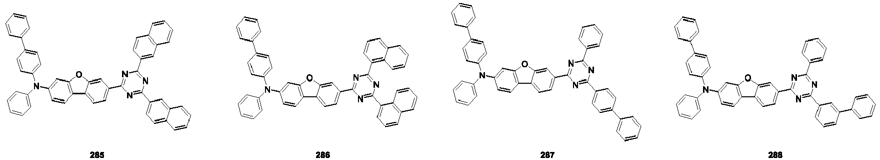

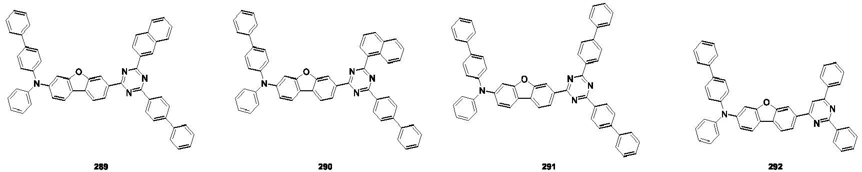

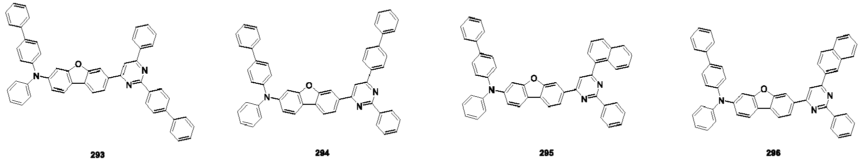

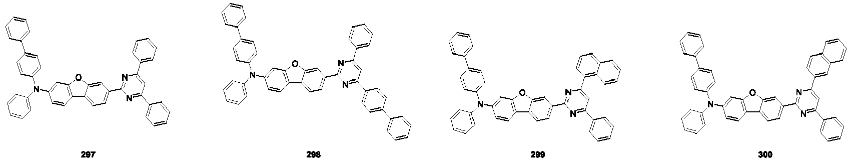

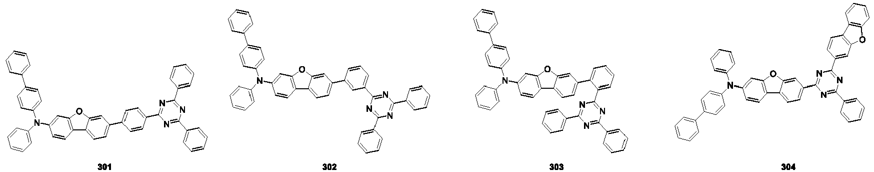

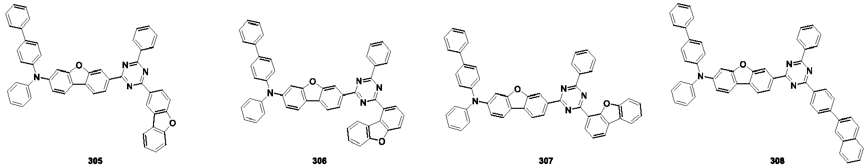

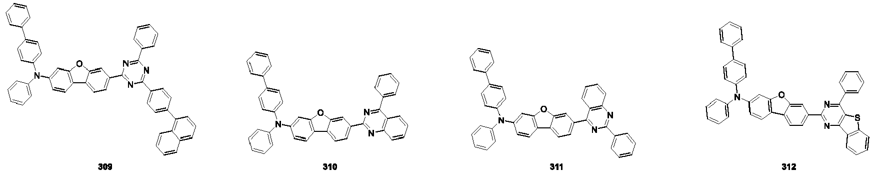

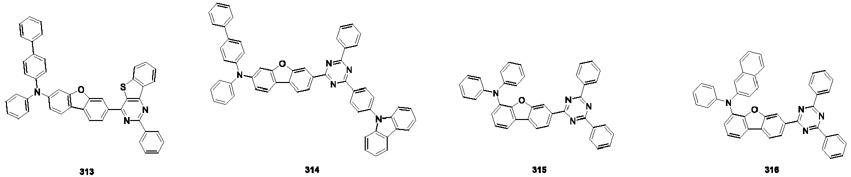

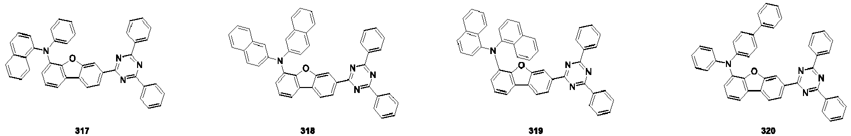

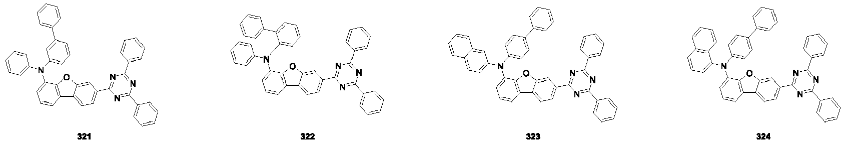

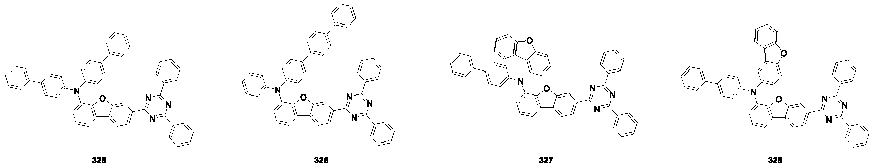

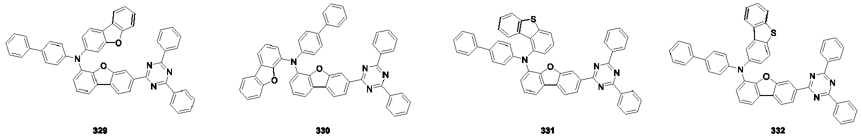

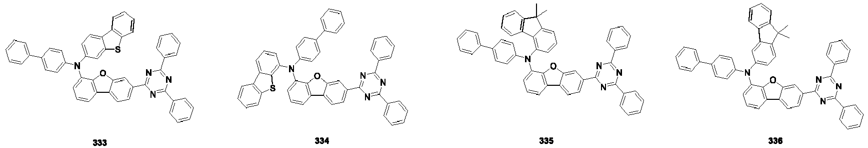

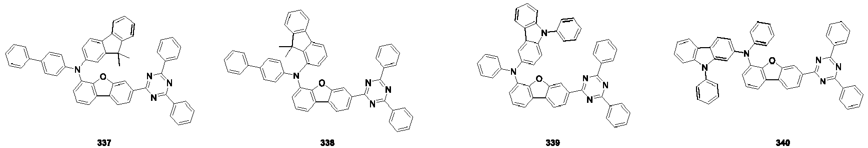

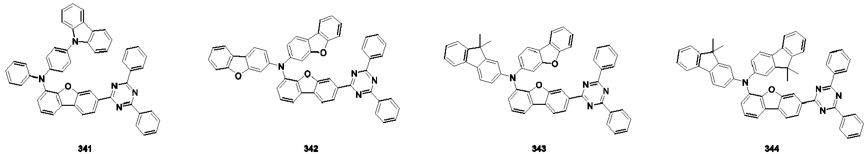

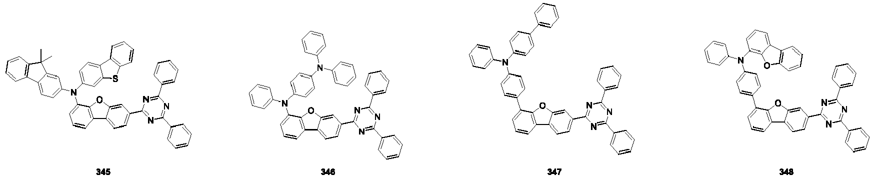

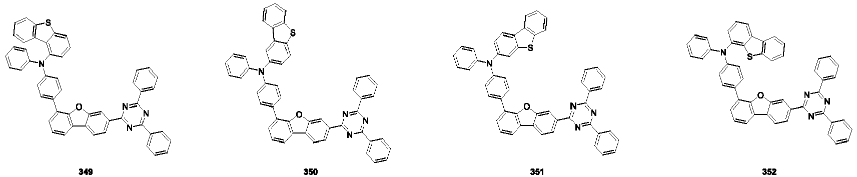

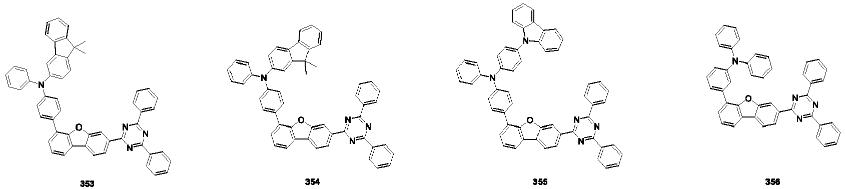

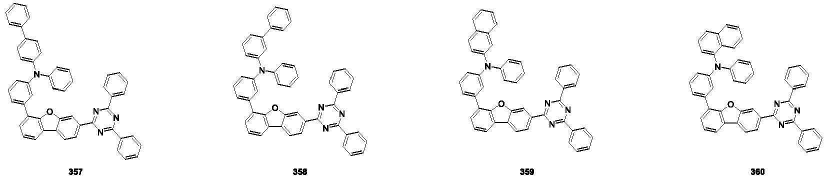

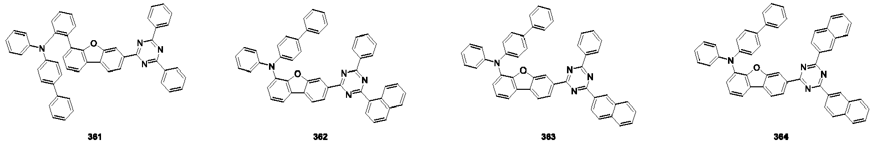

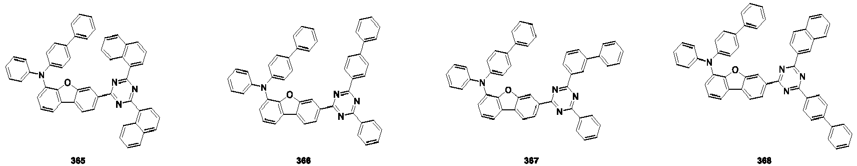

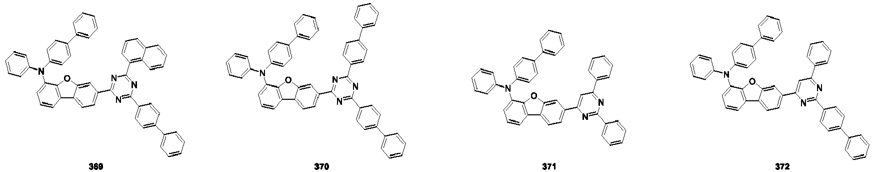

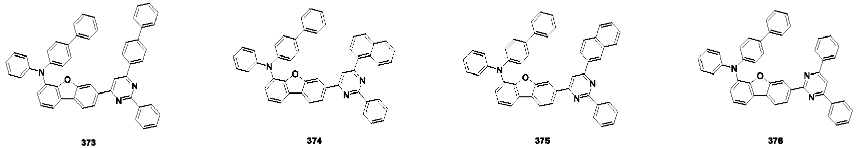

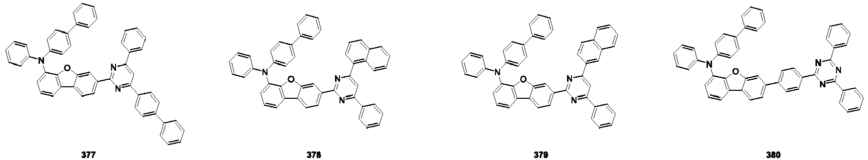

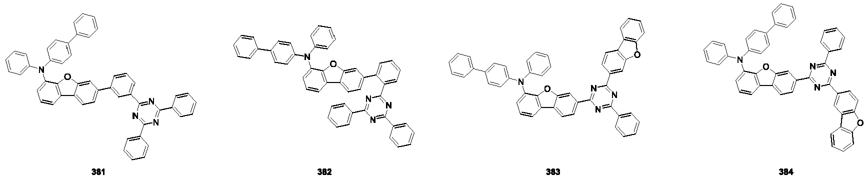

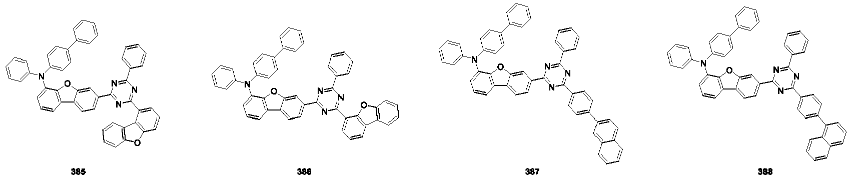

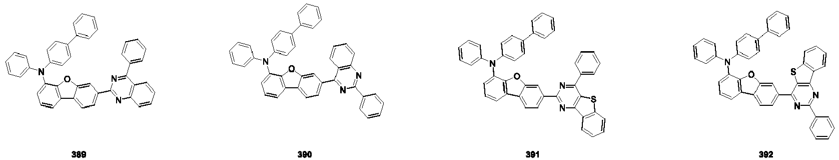

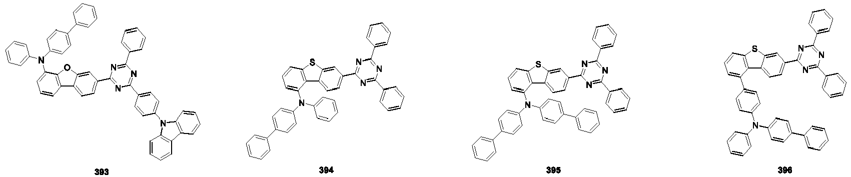

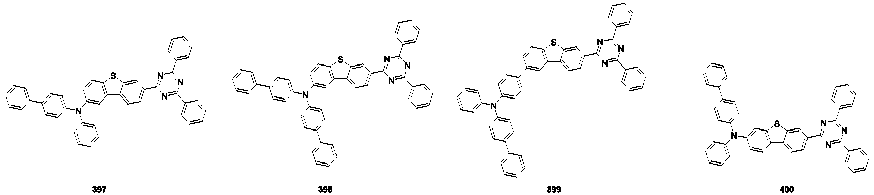

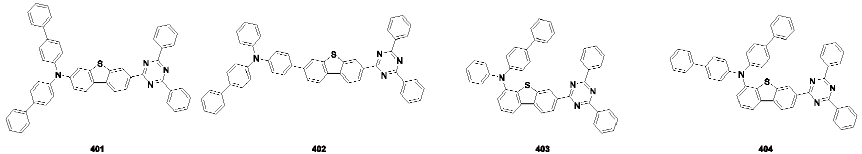

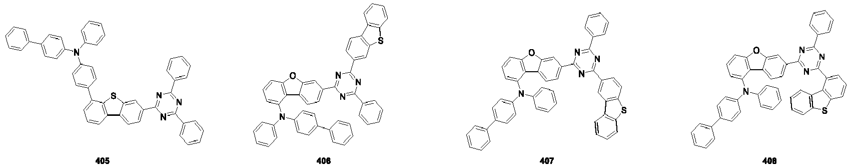

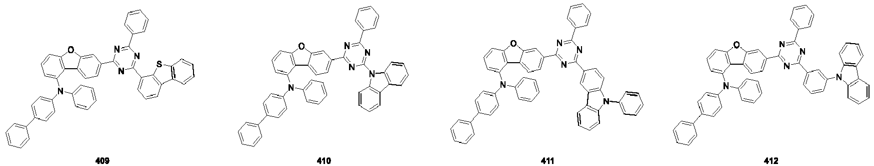

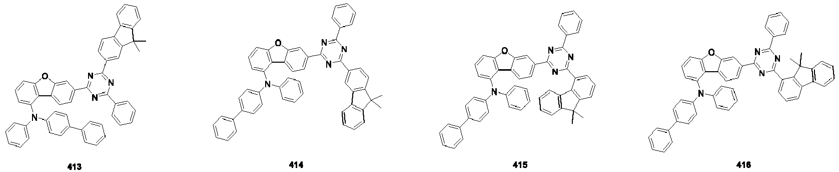

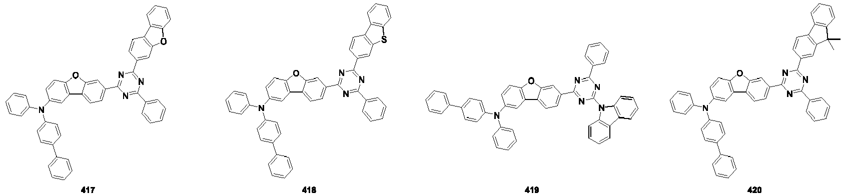

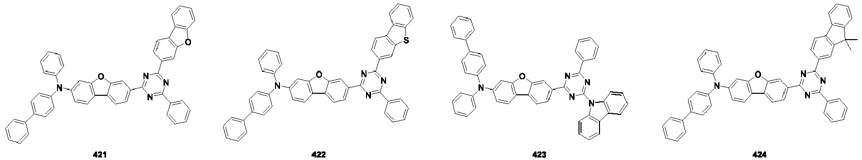

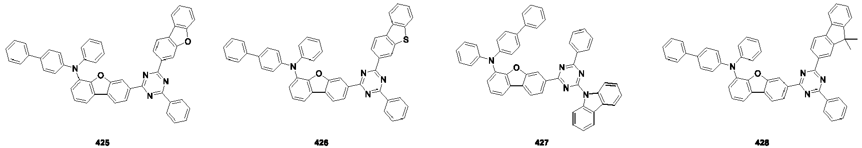

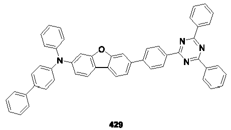

7. An organic light emitting device comprising:
a first electrode;
a second electrode provided opposite to the first electrode; and
one or more organic material layers provided between the first electrode and the second electrode,
wherein one or more layers of the organic material layers comprise one or more types of the heterocyclic compound of any one of Claims 1 to 6.

8. The organic light emitting device of Claim 7, wherein the organic material layer comprises a light emitting layer, and the light emitting layer comprises the heterocyclic compound.

9. The organic light emitting device of Claim 7, wherein the organic material layer comprises an electron injection layer or an electron transfer layer, and the electron injection layer or the electron transfer layer comprises the heterocyclic compound.

10. The organic light emitting device of Claim 7, wherein the organic material layer comprises an electron blocking layer or a hole blocking layer, and the electron blocking layer or the hole blocking layer comprises the heterocyclic compound.

11. The organic light emitting device of Claim 7, wherein organic material layer comprises a hole transfer layer, and the hole transfer layer comprises the heterocyclic compound.

12. The organic light emitting device of Claim 7, further comprising one, two or more layers selected from the group consisting of a light emitting layer, a hole injection layer, a hole transfer layer, an electron injection layer, an electron transfer layer, an electron blocking layer and a hole blocking layer.
